# EUROPEAN PATENT APPLICATION

(11) **EP 4 299 591 A1**
(43) Date of publication of application: **03.01.2024**
(21) Application number: 22758847.2
(22) Date of filing: 22.02.2022
(51) Int. Cl.: C07K 16/28, C12N 15/13, A61K 39/395, A61P 35/00

(54) **PREPARATION OF SIGLEC-15 BINDING PROTEIN AND USE THEREOF**

(30) Priority: 23.02.2021 CN 202110202065
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: YANG, Xinxiu, Shanghai 201203 (CN); GU, Chunyin, Shanghai 201203 (CN); WANG, Zongda, Shanghai 201203 (CN); CAO, Xiaodan, Shanghai 201203 (CN); LIU, Xiaowu, Shanghai 201203 (CN); LIU, Peipei, Shanghai 201203 (CN); YANG, Lu, Shanghai 201203 (CN); DENG, Sujun, Shanghai 201203 (CN); PAN, Zhongzong, Shanghai 201203 (CN); WANG, Xueping, Shanghai 201203 (CN)
(74) Representative: Sagittarius IP
(86) International application number: PCT/CN2022/077193
(87) International publication number: WO 2022/179483

(57) **Abstract**

The present invention provides a preparation of a Siglec-15 binding protein and the use thereof, and specifically relates to an isolated antigen-binding protein, which comprises HCDR3. Provided is the use of the antigen-binding protein in the prevention and treatment of diseases.

## Description

### FIELD OF THE INVENTION

The present application relates to the field of biomedicine, and in particular to a preparation of a Siglec-15 binding protein and use thereof.

### BACKGROUND OF THE INVENTION

As a class of classical immunoglobulin-like lectins, Siglecs (sialic acid-binding immunoglobulin-like lectins) can regulate immunologic balance in vivo. There are as many as 30 members in the Siglec family, which are expressed on the cell surface. Abnormalities of multiple molecules in this family member are associated with many diseases, with Siglec-15 being one of the members. Siglec-15 contains no immunoreceptor tyrosine-based inhibitiory motifs (ITIMs) or ITIM-like sequences in the intracellular region, intracellular Siglec-15 is associated with DAP12 or DAP10, both of which contain immunoreceptor tyrosine-based activating motifs (ITAMs) signals, as indicated by the experimental data. However, most of the intracellular segments of Siglec contain immunoreceptor tyrosine-based inhibitiory motifs, thereby exerting immunosuppressive functions. It has been shown that Siglec-15 can bind to sialyl-Tn and Neu5 Aca2-6GalNAca, suggesting that it can be a ligand for Siglec-15. Siglec-15 is predominantly expressed on myeloid cells in normal humans. Siglec-15 is abnormally highly expressed in a variety of solid tumors. Preclinical in vitro experiments and animal tumor models indicate that Siglec-15 antibody can activate T cells and inhibit tumors. However, there is currently a lack of suitable Siglec-15 antibodies for tumor therapy, and there is an urgent need in the art to develop more Siglec-15 binding proteins with therapeutic effects.

### SUMMARY OF THE INVENTION

The present application provides a Siglec-15 binding protein. The Siglec-15 binding protein of the present application can have one or more effects selected from the group consisting of: (1) having the ability to bind to Siglec-15 or a functional fragment thereof with a K_{D} value of about 1.56E-10 M or less; (2) having the ability to bind to Siglec-15 or a functional fragment thereof with an EC₅₀ value of about 3.25 nM or less; (3) having good physicochemical properties, such as thermal stability; (4) having the ability to affect the immune cells proliferation, e.g., to reverse the immune cell proliferation inhibition caused by Siglec-15; (5) having tumor inhibition ability, e.g., can inhibit a tumor with a tumor volume inhibition rate of about 23% or more; (6) having the ability to affect the cytokines secretion.

In one aspect, the present application provides an isolated antigen-binding protein comprising HCDR3 comprising an amino acid sequence as set forth in SEQ ID NO: 1.

In some embodiments, the isolated antigen-binding protein comprises HCDR2 comprising an amino acid sequence as set forth in SEQ ID NO: 33.

In some embodiments, the isolated antigen-binding protein comprises HCDR2 comprising an amino acid sequence as set forth in SEQ ID NO: 2, SEQ ID NO: 3, SEQ ID NO: 4, SEQ ID NO: 5, or SEQ ID NO: 6.

In some embodiments, the isolated antigen-binding protein comprises HCDR1 comprising an amino acid sequence as set forth in SEQ ID NO: 34.

In some embodiments, the isolated antigen-binding protein comprises HCDR1 comprising an amino acid sequence as set forth in SEQ ID NO: 7 or SEQ ID NO: 8.

In some embodiments, the isolated antigen-binding protein comprises LCDR3 comprising an amino acid sequence as set forth in SEQ ID NO: 9.

In some embodiments, the isolated antigen-binding protein comprises LCDR2 comprising an amino acid sequence as set forth in SEQ ID NO: 35.

In some embodiments, the isolated antigen-binding protein comprises LCDR2 comprising an amino acid sequence as set forth in SEQ ID NO: 10, SEQ ID NO: 11, or SEQ ID NO: 12.

In some embodiments, the isolated antigen-binding protein comprises LCDR1 comprising an amino acid sequence as set forth in SEQ ID NO: 36.

In some embodiments, the isolated antigen-binding protein comprises LCDR1 comprising an amino acid sequence as set forth in SEQ ID NO: 13 or SEQ ID NO: 14.

In some embodiments, the isolated antigen-binding protein comprises H-FR1, wherein the C-terminus of the H-FR1 is directly or indirectly linked to the N-terminus of the HCDR1, and the H-FR1 comprises an amino acid sequence as set forth in SEQ ID NO: 15.

In some embodiments, the isolated antigen-binding protein comprises H-FR2, wherein the H-FR2 is located between the HCDR1 and the HCDR2, and the H-FR2 comprises an amino acid sequence as set forth in SEQ ID NO: 16.

In some embodiments, the isolated antigen-binding protein comprises H-FR3, wherein the H-FR3 is located between the HCDR2 and the HCDR3, and the H-FR3 comprises an amino acid sequence as set forth in SEQ ID NO: 17.

In some embodiments, the isolated antigen-binding protein comprises H-FR4, wherein the N-terminus of the H-FR4 is directly or indirectly linked to the C-terminus of the HCDR3, and the H-FR4 comprises an amino acid sequence as set forth in SEQ ID NO: 18.

In some embodiments, the isolated antigen-binding protein comprises L-FR1, wherein the C-terminus of the L-FR1 is directly or indirectly linked to the N-terminus of the LCDR1, and the L-FR1 comprises an amino acid sequence as set forth in SEQ ID NO: 19.

In some embodiments, the isolated antigen-binding protein comprises L-FR2, wherein the L-FR2 is located between the LCDR1 and the LCDR2, and the L-FR2 comprises an amino acid sequence as set forth in SEQ ID NO: 20.

In some embodiments, the isolated antigen-binding protein comprises L-FR3, wherein the L-FR3 is located between the LCDR2 and the LCDR3, and the L-FR3 comprises an amino acid sequence as set forth in SEQ ID NO: 21.

In some embodiments, the isolated antigen-binding protein comprises L-FR4, wherein the N-terminus of the L-FR4 is directly or indirectly linked to the C-terminus of the LCDR3, and the L-FR4 comprises an amino acid sequence as set forth in SEQ ID NO: 22.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 33, and the HCDR1 comprises an amino acid sequence as set forth in SEQ ID NO: 34.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 2, SEQ ID NO: 3, SEQ ID NO: 4, SEQ ID NO: 5, or SEQ ID NO: 6, and the HCDR1 comprises an amino acid sequence as set forth in SEQ ID NO: 7 or SEQ ID NO: 8.

In some embodiments of the isolated antigen-binding protein, wherein the LCDR3 comprises an amino acid sequence as set forth in SEQ ID NO: 9, the LCDR2 comprises an amino acid sequence as set forth in SEQ ID NO: 35, and the LCDR1 comprises an amino acid sequence as set forth in SEQ ID NO: 36.

In some embodiments of the isolated antigen-binding protein, wherein the LCDR3 comprises an amino acid sequence as set forth in SEQ ID NO: 9, the LCDR2 comprises an amino acid sequence as set forth in SEQ ID NO: 10, SEQ ID NO: 11, or SEQ ID NO: 12, and the HCDR1 comprises an amino acid sequence as set forth in SEQ ID NO: 13 or SEQ ID NO: 14.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 33, the HCDR1 comprises an amino acid sequence as set forth in SEQ ID NO: 34, the LCDR3 comprises an amino acid sequence as set forth in SEQ ID NO: 9, the LCDR2 comprises an amino acid sequence as set forth in SEQ ID NO: 35, and the LCDR1 comprises an amino acid sequence as set forth in SEQ ID NO: 36.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 2, SEQ ID NO: 3, SEQ ID NO: 4, SEQ ID NO: 5, or SEQ ID NO: 6, the HCDR1 comprises an amino acid sequence as set forth in SEQ ID NO: 7 or SEQ ID NO: 8, the LCDR3 comprises an amino acid sequence as set forth in SEQ ID NO: 9, the LCDR2 comprises an amino acid sequence as set forth in SEQ ID NO: 10, SEQ ID NO: 11, or SEQ ID NO: 12, and the LCDR1 comprises an amino acid sequence as set forth in SEQ ID NO: 13 or SEQ ID NO: 14.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 2, the HCDR1 comprises an amino acid sequence as set forth in SEQ ID NO: 7, the LCDR3 comprises an amino acid sequence as set forth in SEQ ID NO: 9, the LCDR2 comprises an amino acid sequence as set forth in SEQ ID NO: 10, and the LCDR1 comprises an amino acid sequence as set forth in SEQ ID NO: 13.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 2, the HCDR1 comprises an amino acid sequence as set forth in SEQ ID NO: 8, the LCDR3 comprises an amino acid sequence as set forth in SEQ ID NO: 9, the LCDR2 comprises an amino acid sequence as set forth in SEQ ID NO: 10, and the LCDR1 comprises an amino acid sequence as set forth in SEQ ID NO: 13.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 3, the HCDR1 comprises an amino acid sequence as set forth in SEQ ID NO: 7, the LCDR3 comprises an amino acid sequence as set forth in SEQ ID NO: 9, the LCDR2 comprises an amino acid sequence as set forth in SEQ ID NO: 10, and the LCDR1 comprises an amino acid sequence as set forth in SEQ ID NO: 13.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 4, the HCDR1 comprises an amino acid sequence as set forth in SEQ ID NO: 7, the LCDR3 comprises an amino acid sequence as set forth in SEQ ID NO: 9, the LCDR2 comprises an amino acid sequence as set forth in SEQ ID NO: 10, and the LCDR1 comprises an amino acid sequence as set forth in SEQ ID NO: 13.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 5, the HCDR1 comprises an amino acid sequence as set forth in SEQ ID NO: 7, the LCDR3 comprises an amino acid sequence as set forth in SEQ ID NO: 9, the LCDR2 comprises an amino acid sequence as set forth in SEQ ID NO: 10, and the LCDR1 comprises an amino acid sequence as set forth in SEQ ID NO: 13.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 6, the HCDR1 comprises an amino acid sequence as set forth in SEQ ID NO: 7, the LCDR3 comprises an amino acid sequence as set forth in SEQ ID NO: 9, the LCDR2 comprises an amino acid sequence as set forth in SEQ ID NO: 10, and the LCDR1 comprises an amino acid sequence as set forth in SEQ ID NO: 13.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 2, the HCDR1 comprises an amino acid sequence as set forth in SEQ ID NO: 7, the LCDR3 comprises an amino acid sequence as set forth in SEQ ID NO: 9, the LCDR2 comprises an amino acid sequence as set forth in SEQ ID NO: 10, and the LCDR1 comprises an amino acid sequence as set forth in SEQ ID NO: 14.

In some embodiments of the isolated antigen-binding protein wherein the HCDR3 comprises an amino acid sequence as set forth in SEQ ID NO: 1, HCDR2 comprises an amino acid sequence as set forth in SEQ ID NO: 3, HCDR1 comprises an amino acid sequence as set forth in SEQ ID NO: 7, LCDR3 comprises an amino acid sequence as set forth in SEQ ID NO: 9, LCDR2 comprises an amino acid sequence as set forth in SEQ ID NO: 11, and LCDR1 comprises an amino acid sequence as set forth in SEQ ID NO: 13.

In some embodiments of the isolated antigen-binding protein, wherein the HCDR3 comprises an amino acid sequence as set forth in SEQ ID NO: 1, the HCDR2 comprises an amino acid sequence as set forth in SEQ ID NO: 3, the HCDR1 comprises an amino acid sequence as set forth in SEQ ID NO: 7, the LCDR3 comprises an amino acid sequence as set forth in SEQ ID NO: 9, the LCDR2 comprises an amino acid sequence as set forth in SEQ ID NO: 12, and the LCDR1 comprises an amino acid sequence as set forth in SEQ ID NO: 13.

In some embodiments, the isolated antigen-binding protein comprises a heavy chain variable region (VH) comprising an amino acid sequence as set forth in SEQ ID NO: 37.

In some embodiments, the isolated antigen-binding protein comprises VH comprising an amino acid sequence as set forth in SEQ ID NO: 23, SEQ ID NO: 24, SEQ ID NO: 25, SEQ ID NO: 26, SEQ ID NO: 27, or SEQ ID NO: 28.

In some embodiments, the isolated antigen-binding protein comprises a light chain variable region (VL) comprising an amino acid sequence as set forth in SEQ ID NO: 38.

In some embodiments, the isolated antigen-binding protein comprises VL comprising an amino acid sequence as set forth in SEQ ID NO: 29, SEQ ID NO: 30, SEQ ID NO: 31, or SEQ ID NO: 32.

In some embodiments, the isolated antigen-binding protein comprises VH comprising an amino acid sequence as set forth in SEQ ID NO: 37 and VL comprising an amino acid sequence as set forth in SEQ ID NO: 38.

In some embodiments, the isolated antigen-binding protein comprises VH comprising an amino acid sequence as set forth in SEQ ID NO: 23, SEQ ID NO: 24, SEQ ID NO: 25, SEQ ID NO: 26, SEQ ID NO: 27, or SEQ ID NO: 28; and VL comprising an amino acid sequence as set forth in SEQ ID NO: 29, SEQ ID NO: 30, SEQ ID NO: 31, or SEQ ID NO: 32.

In some embodiments, the isolated antigen-binding protein comprises VH comprising an amino acid sequence as set forth in SEQ ID NO: 23 and VL comprising an amino acid sequence as set forth in SEQ ID NO: 29.

In some embodiments, the isolated antigen-binding protein comprises VH comprising an amino acid sequence as set forth in SEQ ID NO: 24 and VL comprising an amino acid sequence as set forth in SEQ ID NO: 29.

In some embodiments, the isolated antigen-binding protein comprises VH comprising an amino acid sequence as set forth in SEQ ID NO: 25 and VL comprising an amino acid sequence as set forth in SEQ ID NO: 29.

In some embodiments, the isolated antigen-binding protein comprises VH comprising an amino acid sequence as set forth in SEQ ID NO: 26 and VL comprising an amino acid sequence as set forth in SEQ ID NO: 29.

In some embodiments, the isolated antigen-binding protein comprises VH comprising an amino acid sequence as set forth in SEQ ID NO: 27 and VL comprising an amino acid sequence as set forth in SEQ ID NO: 29.

In some embodiments, the isolated antigen-binding protein comprises VH comprising an amino acid sequence as set forth in SEQ ID NO: 28 and VL comprising an amino acid sequence as set forth in SEQ ID NO: 29.

In some embodiments, the isolated antigen-binding protein comprises VH comprising an amino acid sequence as set forth in SEQ ID NO: 23 and VL comprising an amino acid sequence as set forth in SEQ ID NO: 30.

In some embodiments, the isolated antigen-binding protein comprises VH comprising an amino acid sequence as set forth in SEQ ID NO: 25 and VL comprising an amino acid sequence as set forth in SEQ ID NO: 31.

In some embodiments, the isolated antigen-binding protein comprises VH comprising an amino acid sequence as set forth in SEQ ID NO: 25 and VL comprising an amino acid sequence as set forth in SEQ ID NO: 32.

In some embodiments, the isolated antigen-binding protein comprises an antibody heavy chain constant region.

In some embodiments of the isolated antigen-binding protein, wherein the antibody heavy chain constant region comprises a heavy chain constant region from a human antibody.

In some embodiments of the isolated antigen-binding protein, wherein the antibody heavy chain constant region comprises a heavy chain constant region from IgG.

In some embodiments of the isolated antigen-binding protein, wherein the antibody heavy chain constant region comprises a heavy chain constant region from IgG1.

In some embodiments, the isolated antigen-binding protein comprises an antibody light chain constant region.

In some embodiments of the isolated antigen-binding protein, wherein the antibody light chain constant region comprises a light chain constant region from a human antibody.

In some embodiments of the isolated antigen-binding protein, wherein the antibody light chain constant region comprises a constant region from human Igκ.

In some embodiments, the isolated antigen-binding protein includes an antibody or antigen-binding fragment thereof.

In some embodiments of the isolated antigen-binding protein, wherein the antibody includes a monoclonal antibody.

In some embodiments of the isolated antigen-binding protein, wherein the antibody is selected from one or more of the groups consisting of a murine antibody, a chimeric antibody, a humanized antibody, and a fully human antibody.

In some embodiments of the isolated antigen-binding protein, wherein the antigen-binding fragment is selected from one or more of the groups consisting of Fab, Fab', a Fv fragment, F(ab')₂, F(ab)₂, scFv, di-scFv, VHH, and dAb.

In some embodiments, the isolated antigen-binding protein is capable of specifically binding to a sialic acid-binding immunoglobulin-like lectin (Siglec) or a functionally active fragment thereof.

In some embodiments of the isolated antigen-binding protein, wherein the Siglec includes Siglec-15.

In some embodiments of the isolated antigen-binding protein, wherein the Siglec includes human Siglec, monkey Siglec, and/or murine Siglec.

In some embodiments, the isolated antigen-binding protein has the ability to bind to Siglec-15 or a functional fragment thereof with a K_{D} value of about 1.56E-10 M or less.

In some embodiments, the isolated antigen-binding protein has the ability to bind to Siglec-15 or a functional fragment thereof with an EC₅₀ value of about 3.25 nM or less.

In some embodiments of the isolated antigen-binding protein, wherein the Siglec-15 or functional fragment thereof includes Siglec-15 or functional fragment thereof expressed on a cell.

In some embodiments, the isolated antigen-binding protein has the ability to affect the immune cells proliferation.

In some embodiments of the isolated antigen-binding protein, wherein the affecting immune cell proliferation includes reducing the Siglec-15-associated immune cell proliferation inhibition.

In some embodiments of the isolated antigen-binding protein, wherein the immune cells include immune cells derived from human.

In some embodiments of the isolated antigen-binding protein, wherein the immune cells include lymphocytes.

In some embodiments of the isolated antigen-binding protein, wherein the immune cells include peripheral blood lymphocytes.

In some embodiments of the isolated antigen-binding protein, wherein the immune cells include T cells.

In some embodiments of the isolated antigen-binding protein, wherein the immune cells include CD4⁺ cells and/or CD8⁺ cells.

In some embodiments, the isolated antigen-binding protein has tumor inhibition ability.

In some embodiments of the isolated antigen-binding protein, wherein the tumor inhibition ability includes affecting the volume of the tumor.

In some embodiments, the isolated antigen-binding protein has the ability to inhibit a tumor with a tumor volume inhibition rate of about 23% or more.

In some embodiments, the isolated antigen-binding protein has the ability to affect the cytokine secretion.

In some embodiments of the isolated antigen-binding protein, wherein the secretion includes in vivo, ex vivo and/or in vitro secretion.

In some embodiments of the isolated antigen-binding protein, wherein the cytokine secretion includes comprises lipopolysaccharide-induced cytokine secretion.

In some embodiments of the isolated antigen-binding protein, wherein the cytokine includes an inflammatory factor.

In some embodiments of the isolated antigen-binding protein, wherein the cytokine includes TNF-α and/or IL-6.

In another aspect, the present application also provides a polypeptide comprising the isolated antigen-binding proteins of the present application.

In some embodiments, the polypeptide comprises a fusion protein.

In another aspect, the present application also provides a nucleic acid molecule encoding the isolated antigen-binding protein of the present application and/or the polypeptide of the present application.

In another aspect, the present application also provides a vector comprising the nucleic acid molecule of the present application.

In another aspect, the present application also provides an immunoconjugate comprising the isolated antigen-binding protein of the present application and/or the polypeptide of the present application.

In another aspect, the present application also provides a cell comprising and/or expressing the isolated antigen-binding protein of the present application, comprising and/or expressing the polypeptide of the present application, comprising the nucleic acid molecule of the present application, comprising the vector of the present application and/or comprising the immunoconjugate of the present application.

In another aspect, the present application also provides a pharmaceutical composition comprising the isolated antigen-binding protein of the present application, the polypeptide of the present application, the nucleic acid molecule of the present application, the vector of the present application, the immunoconjugate of the present application, and/or the cell of the present application, and optionally a pharmaceutically acceptable vehicle.

In another aspect, the present application also provides a pharmaceutical combination comprising the isolated antigen-binding protein of the present application, the polypeptide of the present application, the nucleic acid molecule of the present application, the vector of the present application, the immunoconjugate of the present application, the cell of the present application, and/or the pharmaceutical composition of the present application.

In another aspect, the present application also provides a kit comprising the isolated antigen-binding protein of the present application, the polypeptide of the present application, the nucleic acid molecule of the present application, the vector of the present application, the immunoconjugate of the present application, the cell of the present application, the pharmaceutical composition of the present application, and/or the pharmaceutical combination of the present application.

In some embodiments, the kit is used for detecting the presence and/or amount of Siglec in a sample.

In some embodiments of the kit, wherein the Siglec includes Siglec-15.

In another aspect, the present application also provides a method of preparing the antigen-binding protein and/or polypeptide of the present application, comprising culturing the cell of the present application under conditions capable of expressing the antigen-binding protein and/or the polypeptide.

In some embodiments, the method comprises culturing the cell under conditions capable of expressing the antigen-binding protein.

In some embodiments, the method comprises culturing the cell under conditions capable of expressing the polypeptide.

In another aspect, the present application also provides a method of detecting Siglec in a sample, comprising administering the isolated antigen-binding protein of the present application, administering the polypeptide of the present application, administering the nucleic acid molecule of the present application, administering the vector of the present application, administering the immunoconjugate of the present application, administering the cell of the present application, administering the pharmaceutical composition of the present application, administering the pharmaceutical combination of the present application, and/or using the kit of the present application.

In some embodiments of the method, wherein the Siglec includes Siglec-15.

In another aspect, the present application also provides a method of affecting binding of a Siglec or a functionally active fragment thereof to its ligand, comprising administering the isolated antigen-binding protein of the present application, administering the polypeptide of the present application, administering the nucleic acid molecule of the present application, administering the vector of the present application, administering the immunoconjugate of the present application, administering the cell of the present application, administering the pharmaceutical composition of the present application, administering the pharmaceutical combination of the present application, and/or using the kit of the present application.

In some embodiments, the method includes in vivo, ex vivo and/or in vitro methods.

In some embodiments of the method, wherein the Siglec or functionally active fragment thereof includes Siglec-15 or a functionally active fragment thereof.

In some embodiments of the method, wherein the ligand for Siglec includes Leucine-rich repeat-containing protein 4C and/or sialylated Tn (sialyl-Tn).

In another aspect, the present application also provides a method of affecting immune cells proliferation, comprising administering the isolated antigen-binding protein of the present application, administering the polypeptide of the present application, administering the nucleic acid molecule of the present application, administering the vector of the present application, administering the immunoconjugate of the present application, administering the cell of the present application, administering the pharmaceutical composition of the present application, administering the pharmaceutical combination of the present application, and/or using the kit of the present application.

In some embodiments, the method includes in vivo, ex vivo and/or in vitro methods.

In some embodiments of the method, wherein the affecting immune cell proliferation includes reducing the Siglec-15-associated immune cell proliferation inhibition.

In some embodiments of the method, wherein the immune cells include immune cells derived from human.

In some embodiments of the method, wherein the immune cells include lymphocytes.

In some embodiments of the method, wherein the immune cells include peripheral blood lymphocytes.

In some embodiments of the method, wherein the immune cells include T cells.

In some embodiments of the method, wherein the immune cells include CD4⁺ and/or CD8⁺ cells.

In another aspect, the present application also provides a method of affecting cytokine secretion, comprising administering the isolated antigen-binding protein of the present application, administering the polypeptide of the present application, administering the nucleic acid molecule of the present application, administering the vector of the present application, administering the immunoconjugate of the present application, administering the cell of the present application, administering the pharmaceutical composition of the present application, administering the pharmaceutical combination of the present application, and/or using the kit of the present application.

In some embodiments of the method, wherein the secretion includes in vivo, ex vivo and/or in vitro secretion.

In some embodiments of the method, wherein the cytokine secretion includes lipopolysaccharide-associated cytokine secretion.

In some embodiments of the method, wherein the cytokine includes an inflammatory factor.

In some embodiments of the method, wherein the cytokine includes TNF-α and/or IL-6.

In another aspect, the present application also provides the use of the isolated antigen-binding protein of the present application, the polypeptide of the present application, the nucleic acid molecule of the present application, the vector of the present application, the immunoconjugate of the present application, the cell of the present application, the pharmaceutical composition of the present application, and/or the pharmaceutical combination of the present application in the manufacture of a kit.

In some embodiments of the use, wherein the kit is used for detecting the presence and/or amount of Siglec in a sample.

In some embodiments of the use, wherein the Siglec includes Siglec-15.

In another aspect, the present application also provides the use of the isolated antigen-binding protein of the present application, the polypeptide of the present application, the nucleic acid molecule of the present application, the vector of the present application, an immunoconjugate of the present application, the cell of the present application, the pharmaceutical composition of the present application, the pharmaceutical combination of the present application, and/or the kit of the present application in the manufacture of a medicament for the prevention and/or treatment of a disease and/or condition.

In some embodiments of the use, wherein the disease and/or condition includes a tumor.

In some embodiments of the use, wherein the tumor includes a Siglec-associated tumor.

In some embodiments of the use, wherein the Siglec includes Siglec-15.

In some embodiments of the use, wherein the disease and/or condition includes a solid tumor.

In some embodiments of the use, wherein the disease and/or condition includes colon cancer.

In another aspect, the present application also provides the isolated antigen-binding protein of the present application, the polypeptide of the present application, the nucleic acid molecule of the present application, the vector of the present application,the immunoconjugate of the present application, the cell of the present application, the pharmaceutical composition of the present application, the pharmaceutical combination of the present application, and/or the kit of the present application for use in the prevention and/or treatment of a disease and/or condition.

In some embodiments, wherein the disease and/or condition includes a tumor.

In some embodiments, the tumor includes a Siglec-associated tumor.

In some embodiments, the Siglec includes Siglec-15.

In some embodiments, the disease and/or disorder comprises a solid tumor.

In some embodiments, the disease and/or disorder comprises colon cancer.

In another aspect, the present application also provides a method for the prevention and/or treatment of a disease and/or condition, comprising administering to a subject in need thereof the isolated antigen-binding protein of the present application, the polypeptide of the present application, the nucleic acid molecule of the present application, the vector of the present application, the immunoconjugate of the present application, the cell of the present application, the pharmaceutical composition of the present application, the pharmaceutical combination of the present application, and/or the kit of the present application.

In some embodiments, wherein the disease and/or condition includes a tumor.

In some embodiments, the tumor includes a Siglec-associated tumor.

In some embodiments, the Siglec includes Siglec-15.

In some embodiments, the disease and/or disorder comprises a solid tumor.

In some embodiments, the disease and/or disorder comprises colon cancer.

Other aspects and advantages of the present application will become readily apparent to those skilled in the art from the following detailed description. Only exemplary embodiments of the present application are shown and described in the following detailed description. As will be recognized by those skilled in the art, the contents of the present application enable those skilled in the art to make modifications to the specific embodiments disclosed without departing from the spirit and scope of the present application to which the present application pertains. Accordingly, the drawings and description herein are to be regarded as illustrative in nature, and not as restrictive.

### BRIEF DESCRIPTION OF THE DRAWINGS

Specific features of the application to which the present application relates are set forth in the appended claims. The features and advantages of the present application to which the present application relates will be better understood by reference to the exemplary embodiments and drawings described in detail below. A brief description of the drawings is as follows:
Fig. 1 shows a schematic diagram of a construction method of the yeast display library of the present application.
Fig. 2 shows the results for the detection for binding of a Siglec-15 binding protein of the present application to human Siglec-15 on the surface of a cell.
Fig. 3 shows the results for the detection for binding of a Siglec-15 binding protein of the application to monkey Siglec-15 on the surface of a cell.
Fig. 4 shows the results for the detection for binding of a Siglec-15 binding protein of the application to murine Siglec-15 on the surface of a cell.
Fig. 5 shows the results for the detection for reversing the proliferation inhibition effect of Siglec-15 protein on CD4⁺T cells by Siglec-15 binding protein of the present application.
Fig. 6 shows the results for the detection for reversing the proliferation inhibition effect of Siglec-15 protein on CD8⁺T cells by Siglec-15 binding protein of the present application.
Fig. 7 shows the results for the detection for inhibitory effect on tumor growth by Siglec-15 binding proteins of the present application.
Fig. 8 shows the results for the detection for affecting secretion level of TNF-α (TNF-alpha) by Siglec-15 binding proteins of the present application 2 h after LPS injection.
Fig. 9 shows the results for the detection for affecting secretion level of TNF-α (TNF-alpha) by Siglec-15 binding proteins of the present application 6 h after LPS injection.
Fig. 10 shows the results for the detection for affecting secretion level of IL-6 by Siglec-15 binding proteins of the present application 2 h after LPS injection.
Fig. 11 shows the results for the detection for affecting secretion level of IL-6 by Siglec-15 binding proteins of the present application 6 h after LPS injection.

### DETAILED DESCRIPTION OF THE SPECIFIC EMBODIMENTS

Other advantages and effects of the present application will become readily apparent to those skilled in the art from the following description of specific embodiments.

### Definition of Terms

In the present application, the term "sialic acid-binding immunoglobulin-like lectin", "Sialic acid-binding Ig-like lectin" or "Siglec" generally refers to an immunoglobulin-like lectin that can have the ability to recognize a sugar structure containing sialic acid. In vitro, Siglec-15 recombinant protein can inhibit the proliferation of human and mouse T cells and the secretion of IFN-γ, and can also inhibit ovalbumin (OVA) antigen-activated OT-1 T cells. Siglec-15 is poorly expressed in macrophages and shows significant up-regulation of expression following stimulation with the macrophage colony-stimulating factor M-CSF. Compared with normal macrophages, Siglec-15 gene knockout macrophages can further induce T cell proliferation, indicating that Siglec-15 can directly inhibit T cell activity. Preclinical in vitro experiments and animal tumor models indicate that Siglec-15 antibody or Siglec-15 binding protein can activate T cells and inhibit tumor. For example, the Siglec can be Siglec-15 that may have a Uniprot accession number Q6ZMC9. The Siglec proteins of the present application may also encompass functionally active fragments thereof, and are not limited to proteins comprising functionally active fragments of Siglec proteins resulting from processing and/or modification that occurs in a cell.

In the present application, the terms "Leucine-rich repeat-containing protein 4C" or "LRRC 4C" are used interchangeably and refer generally to a protein rich in a repeat leucine sequence. LRRC4 can act as a ligand for Siglect-15, binding to and activating Siglec-15. LRRC4C may have a Uniprot accession number Q9HCJ2. The LRRC4C proteins of the present application can also encompass functionally active fragments thereof, and are not limited to proteins comprising functionally active fragments of LRRC4C proteins resulting from processing and/or modification occurring in a cell.

In the present application, the term "sialylated Tn" or "sialyl-Tn" generally refers to a Tn antigen that contains a sialyl group. The Tn antigen can be an N-acetylgalactosamine (GalNAc) oligosaccharide linked to serine or threonine via a glycosidic bond. Sialyl-Tn expression results from activation of abnormal glycosylation pathways and can typically occur in tumor cells. The sialylated Tn proteins of the present application can also encompass functionally active fragments thereof.

In the present application, the term "Lipopolysaccharide" or "LPS" generally refers to a substance consisting of a lipid and a polysaccharide. Lipopolysaccharides are generally components of the outer wall of the cell wall of Gram-negative bacteria. Lipopolysaccharides can promote the synthesis and/or secretion of various cytokines by human cells to elicit immune response while acting on to the human body.

In the present application, the term "functionally active fragment" generally refers to a fragment that has a partial region of a full-length protein or nucleic acid, but retains or partially retains the biological activity or function of the full-length protein or nucleic acid. For example, a functionally active fragment can retain or partially retain the ability of the full-length protein to bind to another molecule. For example, a functionally active fragment of Siglec can retain or partially retain the function of the full-length Siglec to recognize sialic acid-containing saccharide structures and/or to inhibit the function of the immune system.

In the present application, the term "secretion" generally refers to the transfer of an expressed polypeptide or protein to the extracellular environment by a cell. For example, in the present application, it can be meant that the transfer of the expressed cytokines to the extracellular environment by immune cells.

In the present application, the term "cytokine" generally refers to a protein released by one cell population that acts as an intercellular modulator on another cell. The cytokines of the present application can be interleukins (ILs), such as IL-6, tumor necrosis factors such as TNF-α, and other polypeptide factors. In the present application, the cytokines of the present application can encompass functionally active fragments thereof, and can also include proteins from natural sources or from recombinant cell cultures, as well as biologically active equivalents of the native sequence cytokines.

In the present application, the term "inflammatory factor" generally refers to a cytokine that promotes inflammation. The inflammatory factor of the present application may include interleukin IL-6, and/or tumor necrosis factor TNF-α. In the present application, the inflammatory cytokines of the present application can encompass functionally active fragments thereof, and can also include proteins from natural sources or from recombinant cell cultures, as well as biologically active equivalents of the native sequence cytokines.

In the present application, the term "IL-6" generally refers to an interleukin. IL-6 of the present application can promote inflammation. IL-6 can receive a Uniprot accession number P05231. The IL-6 proteins of the present application can also encompass functionally active fragments thereof, and are not limited to proteins comprising functionally active fragments of IL-6 proteins resulting from processing and/or modification occurring in a cell.

In the present application, the term "TNFα" or "TNF-α" generally refers to tumor necrosis factor α. TNF-α can be a major mediator of inflammatory, immunological and pathophysiological responses. TNF-α may include wild-type TNF-α, polymorphic variants of TNF-α, and functional equivalents of TNF-α from various species (e.g., humans, mice, and monkeys). TNF-α can receive a Uniprot accession number P01375. The TNF-α proteins of the present application can also encompass functionally active fragments thereof, and are not limited to proteins comprising functionally active fragments of TNF-α proteins resulting from processing and/or modification occurring in a cell.

In the present application, the term "lymphocyte" generally refers to white blood cells found in blood, lymph and lymphoid tissues. For example, lymphocytes may include any monocytes and/or non-phagocytic leukocytes.

In the present application, the term "immune cell" generally refers to cells involved in an immune response. Immune cells may include T cells, B cells, natural killer (NK) cells, mast cells, and/or phagocytic cells derived from bone marrow.

In the present application, the term "peripheral blood lymphocyte" generally refers to mature lymphocytes that circulate in the blood and are not localized to organs such as the spleen or lymph nodes. Peripheral blood lymphocytes may include T cells, NK cells and/or B cells.

In the present application, the term "T cell" generally refers to cells derived from thymus. T cells that can involve in a variety of cell-mediated immune responses include thymocytes, naive T lymphocytes, immature T lymphocytes, mature T lymphocytes, resting T lymphocytes or activated T lymphocytes. T cell populations can include, but are not limited to, helper T cells (Th; CD4⁺T cells), cytotoxic T cells (CTL; CD8⁺T cells), CD4⁺CD8⁺T cells, CD4⁻CD8⁻T cells or any other subpopulation of T cells.

In the present application, the term "CD4" generally refers to a cluster of differentiation 4 protein expressed by a cell. CD4 can be a co-receptor for T cell recognition antigens and can bind to a non-polypeptide region of an MHC class II molecule. T cells expressing CD4 (CD4⁺T cells or CD4 positive T cells) can differentiate into helper T cells (Th) upon activation and can modulate an immune response. CD4 can receive a Uniprot accession number P01730. The CD4 proteins of the present application can also encompass functionally active fragments thereof, and are not limited to proteins comprising functionally active fragments of CD4 proteins resulting from processing and/or modification that occurs in a cell.

In the present application, the term "CD8" generally refers to a cluster of differentiation 8 protein expressed by a cell. CD8 can be a co-receptor for T cell recognition antigens and can bind to a non-polypeptide region of an MHC class II molecule. T cells expressing CD8 (CD8⁺T cells or CD8 positive T cells) can differentiate into cytotoxic T cells (CTL) upon activation and can specifically kill target cells. CD8 can receive a Uniprot accession number P10966. The CD8 proteins of the present application can also encompass functionally active fragments thereof, and are not limited to proteins comprising functionally active fragments of CD8 proteins resulting from processing and/or modification that occurs in a cell.

In the present application, the term "in vivo" generally refers to an event that occurs in a subject in vivo.

In the present application, the term "in vitro" generally refers to an event that occurs in a subject in vitro.

In the present application, the term "ex vivo" generally refers to an event involving treatment or surgery of cells, tissues and/or organs that have been removed from the body of a subject. In one embodiment, the cells, tissues and/or organs can be returned to the body of the subject by surgery or treatment.

In the present application, the term "EC₅₀ value" or "EC50 value" generally refers to 50% of between the baseline value and the maximum value of the response of a binding substance (e.g., an antibody) in the context of an in vitro or in vivo assay, the half maximal effective concentration Decreased EC₅₀ values can indicate higher drug affinity and efficacy.

In the present application, the term "K_{D} value" or "KD value" generally refers to a dissociation constant, which can be determined by surface plasmon resonance (SPR). In general, in SPR analysis, real-time binding interactions between a ligand (a substance immobilized on a biosensor matrix) and an analyte (a substance in a solution) are measured by SPR using the BIAcore system (Pharmacia Biosensor, Piscataway, NJ). Surface plasma analysis can also be performed by immobilizing the analyte (a substance on a biosensor matrix) and presenting the ligand.

In the present application, the term "tumor inhibition ability" generally refers to any measurable inhibitory ability for tumor cell proliferation in vitro or tumor growth in vivo, e.g., at least 5%, which can be at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or more, such as at least 100% reduction in tumor cell proliferation in vitro or tumor growth in vivo.

In the present application, the term "isolated" generally refers to a substance obtained from a natural state by artificial means. For example, an unisolated polynucleotide or polypeptide naturally occurs in a living animal and the same polynucleotide or polypeptide isolated from its natural state in high purity can be referred to as isolated. The term "isolated" may not exclude the admixture of an artificial or synthetic substance, nor the presence of other impure substances which do not affect the activity of the substance.

In the present application, the term "isolated antigen-binding protein" generally refers to a protein having antigen-binding ability that has been removed from its naturally occurring state. An "isolated antigen-binding protein" of the present application may comprise a portion that binds to an antigen, optionally, a framework or framework portion that allows the portion that binds to an antigen to adopt a conformation that promotes the binding of the antigen-binding portion to the antigen. The antigen-binding proteins can comprise, for example, protein framework regions (FRs) derived from an antibody or alternative protein framework regions or artificial framework regions with grafted CDR or CDR derivatives. Such frameworks can include, but are not limited to, framework regions derived from an antibody comprising mutations introduced, for example, to stabilize the three-dimensional structure of the antigen-binding protein, as well as fully synthetic framework regions comprising, for example, biocompatible polymers. Examples of antigen-binding proteins can include, but are not limited to: a human antibody, a humanized antibody; a chimeric antibody; a recombinant antibody; a single chain antibody; a bifunctional antibody; a trifunctional antibody; a tetrafunctional antibody; Fab, Fab', a Fv fragment, F(ab')2, F(ab)2, scFv, di-scFv, dAb, VHH, an IgD antibody; an IgE antibody; an IgM antibody; an IgG1 antibody; an IgG2 antibody; an IgG3 antibody; and/or an IgG4 antibody and a fragment thereof.

In the present application, the term "CDR", also referred to as "complementarity determining region", generally refers to a region in an antibody variable domain, the sequence of which can be highly variable and/or form a structurally defined loop. For example, an antibody may comprise six CDRs; three in VH (HCDR1, HCDR2, and HCDR3) and three in VL (LCDR1, LCDR2, and LCDR3). In some embodiments, naturally occurring camel antibodies consisting only of heavy chains can function normally and stably in the absence of light chains. CDR in an antibody can be determined by a variety of coding systems, e.g., CCG, Kabat, Chothia, IMGT, comprehensively considering Kabat/Chothia, etc. Such coding systems are known in the art, see, for example, www.bioinf.org.uk/abs/index.html#kabatnum. For example, the numbering for amino acid sequence of the antigen-binding proteins can follow the IMGT numbering scheme (IMGT, IMGT, the international ImMunoGeneTics information system@imgt.cines.fr; imgt.cines.fr; Lefranc et al., 1999, Nucleic Acids Res. 27: 209-212; Ruiz et al.,2000 Nucleic Acids Res. 28: 219-221; Lefranc et al., 2001, Nucleic Acids Res. 29:207-209; Lefranc et al., 2003, Nucleic Acids Res. 31: 307-310; Lefranc et al., 2005, DevComp Immunol 29: 185-203). For example, the CDR of the antigen-binding protein can be determined according to the Kabat numbering system (see, e.g., Kabat EA &Wu TT (1971) Ann NY AcadSci 190:382-391 and Kabat EAet al., (1991) Sequences of Proteins of Immunological Interest, Fifth Edition, U.S. Department of Health and Human Services, NIH Publication No.91-3242).

In the present application, the term "FR" generally refers to a more highly conserved portion of an antibody variable domain, referred to as a framework region. For example, the variable domains of native heavy and light chains can each comprise four FR regions, namely four in VH (H-FR1, H-FR2, H-FR3, and H-FR4) and four in VL (L-FR1, L-FR2, L-FR3, and L-FR4).

In the present application, the terms "variable domain" and "variable region" are used interchangeably and generally refer to a portion of an antibody heavy and/or light chain. The variable domains of the heavy and light chains can be referred to as "VH" and "VL", respectively (or "V_{H}" and "V_{L}", respectively). These domains can generally be the most diverse portions of an antibody (relative to other antibodies of the same type), and may comprise an antigen-binding site. In the present application, the term "variable" generally means that certain segments of the variable domain can differ substantially in sequence between antibodies. A variable domain mediates the antigens binding and determines the specificity of a particular antibody for its particular antigen. However, the variability may not be uniformly distributed throughout the variable domain. It can be typically concentrated in three segments called hypervariable regions (CDRs or HVRs) in the light and heavy chain variable domains. A more highly conserved portion of a variable domain can be referred to as a framework region (FR). The variable domains of native heavy and light chains respectively comprises four FR regions, which are predominantly in a β-sheet configuration and connected by three CDRs to form loops connection, and in some cases to form a portion of the β-sheet structure. The CDRs in each chain can be held together in close proximity by the FR region, and the CDR from the other chain together facilitate the formation of the antigen-binding site of the antibody.

In the present application, the term "antibody" generally refers to an immunoglobulin or fragment or derivative thereof, and encompasses any polypeptide that includes an antigen-binding site, whether produced in vitro or in vivo. The term can include, but is not limited to, a polyclonal antibody, a monoclonal antibody, a monospecific antibody, a multispecific antibody, a non-specific antibody, a humanized antibody, a single-chain antibody, a chimeric antibody, a synthetic antibody, a recombinant antibody, a hybrid antibody, a mutated antibody, and a grafted antibody. Unless otherwise modified by the term "intact", as in "intact antibodies", for purposes of the present invention, the term "antibody" can also include antibody fragments, such as Fab, F(ab')2, Fv, scFv, Fd, VHH, dAb, and other antibody fragments that retain antigen-binding function (e.g., specifically binding to human Siglec). Typically, such fragments may comprise an antigen-binding domain. An elementary 4-chain antibody unit can be a heterotetrameric glycoprotein consisting of two identical light (L) chains and two identical heavy (H) chains. An IgM antibody can consist of five elementary heterotetrameric units combined with another polypeptide called the J chain, and contains ten antigen-binding sites, while an IgA antibody may comprise two to five elementary 4-chain units that can be polymerized in combination with the J chain to form a multivalent combination. In terms of IgG, the 4-chain unit can generally be about 150, 000 daltons. Each L chain can be linked to an H chain via one covalent disulfide bond, while two H chains can be linked to each other by one or more disulfide bonds depending on the H chain isotype. Each of H and L chains can also have regularly spaced intrachain disulfide bridges. Each H chain can have a heavy chain variable region (VH) at the N-terminus, followed by three constant domains (CH) for each of the alpha (α) and gamma (γ) chains, and four CH domains for the mu (µ) and epsilon (ε) isotypes. Each L chain can have a light chain variable region (VL) at the N-terminus and a constant domain at its other end. VL can correspond to VH, and the light chain constant region (CL) can correspond to the first constant domain (CH1) of the heavy chain. A particular amino acid residue can be considered to form an interface between the light and heavy chain variable domains. VH and VL can be paired together to form a single antigen-binding site. The L chains from any vertebrate species can be classified into one of two distinct types, called κ and λ, based on the amino acid sequences of their constant domain. Depending on the amino acid sequence of the constant domain of the heavy chain constant region (CH), immunoglobulins can be classified into different classes or isotypes. There are currently five classes of immunoglobulins: IgA, IgD, IgE, IgG such as IgG1, IgG2, IgG3, and/or IgG4, and IgM, having heavy chains designated as alpha (α), delta (δ), epsilon (ε), gamma (γ), and mu (µ), respectively.

In the present application, the term "antigen-binding fragment" generally refers to one or more fragments that have the ability to specifically bind to an antigen (e.g., Siglec). In the present application, the antigen-binding fragments may include Fab, Fab', F(ab)2, a Fv fragment, F(ab')2, scFv, di-scFv, VHH and/or dAb.

In the present application, the term "Fab" generally refers to an antigen-binding fragment of an antibody. As described above, intact antibodies can be digested with papain. Digestion of antibodies with papain produces two identical antigen-binding fragments, i.e., a "Fab" fragment and a residual "Fc" fragment (i.e., the Fc region). A Fab fragment can consist of one complete L chain with a variable region of one heavy chain and a first constant region (CH1) of the H chain (VH).

In the present application, the term "F(ab)2" generally refers to an antigen-binding fragment of an antibody. For example, F(ab)2 can be linked by two Fab fragments.

In the present application, the term "Fab" generally refers to a monovalent antigen-binding fragment of a human monoclonal antibody, which is slightly larger than a Fab fragment. For example, a Fab' fragment may comprise all light chains, all heavy chain variable regions, and all or a portion of the first and second constant regions of the heavy chain. For example, a Fab' fragment can also include a portion of or all of the 220-330 amino acid residues of the heavy chain.

In the present application, the term "F(ab')2" generally refers to an antibody fragment produced by digestion of an intact antibody with pepsin. The F(ab')2 fragment contains two Fab fragments held together by disulfide bonds and a portion of the hinge region. F(ab')2 fragments have bivalent antigen-binding activity and are capable of cross-linking antigens.

In the present application, the term "Fv fragment" generally refers to a monovalent antigen-binding fragment of a human monoclonal antibody, including all or a portion of a heavy chain variable region and a light chain variable region, and lacking a heavy chain constant region and a light chain constant region. Heavy chain variable regions and light chain variable regions can include, for example, CDR. For example, Fv fragments include all or a portion of the amino-terminal variable region of about 110 amino acids of the heavy and light chains.

In the present application, the term "scFv" generally refers to a fusion protein comprising at least one antibody fragment including the variable region of the light chain and at least one antibody fragment including the variable region of the heavy chain, wherein the variable regions of the light and heavy chain are adjacent (e.g., via a synthetic linker such as a short flexible polypeptide linker) and capable of being expressed as a single chain polypeptide, and wherein the scFv retains the specificity of the intact antibody from which it is derived. Unless otherwise specified, as used in the present application, an scFv can have the VL and VH variable regions described in any order (e.g., relative to the N-terminus and C-terminus of the polypeptide), and an scFv may comprise a VL-linker-VH or may comprise a VH-linker-VL.

In the present application, the term "di-scFv" generally refers to a divalent scFv, which can be, for example, a molecule in which two scFv molecules are conjugated through a short linker.

In the present application, the term "dAb" generally refers to an antigen-binding fragment consisting of a VH domain or a VL domain composition, see, e.g., Ward et al. (Nature,1989Oct 12; 341(6242): 544-6), see Holt et al., Trends Biotechnol., 2003,21(11):484-490.

In the present application, the term "VHH" generally refers to an antibody comprising the variable antigen-binding domain of a heavy chain antibody (see, Vanlandschoot P. et al., 2011, Antiviral Research 92, 389-407). VHH can also be referred to as Nanobody (Nb).

In the present application, the term "monoclonal antibody" generally refers to a preparation of antibody molecules consisting of a single molecule. The monoclonal antibody is typically highly specific for a single antigenic site. Furthermore, unlike conventional polyclonal antibody preparations (which typically have different antibodies directed against different determinants), each monoclonal antibody can be directed against a single determinant on the antigen. In addition to their specificity, the monoclonal antibody has the advantage that they can be synthesized by hybridoma culture, uncontaminated by other immunoglobulins. The modifier "monoclonal" can indicate the character of the antibody as being obtained from a substantially homogeneous population of antibodies, and is not to be construed as requiring production of the antibody by any particular method. For example, the monoclonal antibody used in the present application can be prepared in hybridoma cells, or can be prepared by recombinant DNA methods.

In the present application, the term "chimeric antibody" generally refers to an antibody in which the variable region is derived from one species and the constant region is derived from another species. Generally, the variable region is derived from an antibody ("parent antibody") of an experimental animal such as a rodent, and the constant region is derived from a human antibody, such that the possibility of eliciting an adverse immune response in a human individual by the resulting chimeric antibody is reduced as compared to the parent (e.g., derived from mice) antibody.

In the present application, the term "humanized antibody" generally refers to an antibody in which some or all of the amino acids outside of the CDR of a non-human antibody (e.g., a murine antibody) have been replaced by corresponding amino acids derived from human immunoglobulins. In the CDR, small additions, deletions, insertions, substitutions or modifications of the amino acids can also be allowed, so long as they still retain the ability of the antibody to bind to a specific antigen. The humanized antibody can optionally comprise at least a portion of a constant region of a human immunoglobulin. The "humanized antibody" can reserve the antigen specificity similar to that of the original antibody. The "humanized" form of a non-human antibody (e.g., a murine antibody) can minimally comprise a chimeric antibody derived from a non-human immunoglobulin sequence. In some cases, residues of CDR in a human immunoglobulin (receptor antibody) can be replaced with residues of CDR from a non-human species (donor antibody) (e.g., a mouse, a rat, a rabbit or a non-human primate) with the desired properties, affinity, and/or ability. In some cases, residues of FR region of the human immunoglobulin can be replaced with corresponding non-human residues. In addition, the humanized antibody may comprise an amino acid modification that is not present in the receptor antibody or in the donor antibody. These modifications can be made to further improve the properties of the antibody, such as binding affinity.

In the present application, the term "fully human antibody" generally refers to an antibody comprising only human immunoglobulin protein sequences. The fully human antibodies can contain murine carbohydrate chains if they are produced in mice, in mouse cells, or in hybridomas derived from mouse cells. Similarly, a "murine antibody", "mouse antibody", or "rat antibody" refers to an antibody comprising only mouse or rat immunoglobulin sequences, respectively. The fully human antibodies can be generated in humans, in transgenic animals with human immunoglobulin germline sequences, by phage display or other molecular biological methods. Exemplary techniques that can be used for making antibodies are known in the art.

In the present application, the term "antigen-binding protein" generally refers to a protein comprising a portion that binds to an antigen, and optionally a scaffold or skeleton portion that allows the portion that binds to an antigen to adopt a conformation that promotes the binding of the antigen-binding protein to the antigen. Examples of antigen-binding proteins include, but are not limited to, antibodies, antigen-binding fragments (Fab, Fab', F(ab)2, a Fv fragment, F(ab')2, scFv, di-scFv, VHH, and/or dAb), immunoconjugates, antibody fragments, antibody derivatives, antibody analogs, or fusion proteins, etc., provided that they exhibit the desired antigen-binding activity. An "isolated antigen-binding protein" of the present application may comprise a portion that binds to an antigen, optionally, a scaffold or framework portion that allows the portion that binds to an antigen to adopt a conformation that promotes the binding of the antigen-binding portion to the antigen.

In the present application, the terms "polypeptide molecule" and "polypeptide", and "peptide" are used interchangeably and generally refer to a polymer of amino acid residues. The term "fusion protein" generally refers to a polypeptide having at least two portions covalently linked together. Each portion can be a polypeptide having different properties. The property can be a biological property, such as in vitro or in vivo activity. The property can also be a simple chemical or physical property, such as binding to a target molecule, catalysis of a reaction, etc. The two portions can be directly linked by a single peptide bond or by a peptide linker.

In the present application, the term "nucleic acid molecule" generally refers to an isolated form of nucleotides, deoxyribonucleotides, or ribonucleotides of any length, or analogs isolated from their natural environment or synthesized synthetically.

In the present application, the term "vector" generally refers to a nucleic acid delivery vehicle into which a polynucleotide encoding a protein can be inserted and expressed. Expression of the genetic material element carried by the vector in a host cell can be achieved by transformation, transduction or transfection of the vector into the host cell. A vector can contain multiple elements for controlling the expression. Alternatively, the vector may comprise an origin of replication. The vector can also include components that facilitate the vector to enter into cells.

In the present application, the term "cell" generally refers to a single cell, a cell line, or a cell culture that may be or has been a recipient of a plasmid or vector from a subject, including a nucleic acid molecule of the present application or a vector of the present application. The cell may include a progeny of a single cell. The progeny may not necessarily be identical (in morphology or in genome to the total DNA complement) to the original parent cell due to natural, accidental, or deliberate mutation. The cell may include a cell transfected in vitro with the vectors of the present application.

In the present application, the term "immunoconjugate" generally refers to a conjugate formed by the conjugation of other reagents (e.g., a chemotherapeutic agent, a radioactive element, a cytostatic agent, and a cytotoxic agent) to the antibody or antigen-binding fragment thereof (e.g., via covalent attachment of a linking molecule), wherein the conjugate can deliver the other reagents to a target cell (e.g., a tumor cell) via specific binding of the antibody or antigen-binding fragment thereof to an antigen on the target cell.

In the present application, the term "pharmaceutical composition" generally refers to a composition for the prevention/treatment of a disease or condition. The pharmaceutical composition may comprise an isolated antigen-binding protein of the present application, the nucleic acid molecule of the present application, the vector of the present application, and/or the cell of the present application, and optionally the pharmaceutically acceptable adjuvant. In addition, the pharmaceutical compositions can also comprise suitable preparations such as one or more (pharmaceutically effective) vehicles. Acceptable components of the compositions can be nontoxic to recipients at the dosages and concentrations employed. Pharmaceutical compositions of the present application include, but are not limited to, liquid, frozen, and lyophilized compositions.

In the present application, the term "pharmaceutically acceptable vehicle" generally refers to a pharmaceutically acceptable carrier, excipient, or stabilizer that is non-toxic to the cell or mammal to which it is exposed at the dosages and concentrations employed. Physiologically acceptable carriers may comprise suitable substances. A pharmaceutically acceptable carrier is generally not the same class as a vector used to insert nucleic acids in genetic engineering.

In the present application, the term "solid tumor" generally refers to a tangible tumor that can be detected by means of clinical examination (e.g., X-ray irradiation, CT scan, B-ultrasound or palpation, etc.). The tumors may include neoplasms or solid lesions formed by abnormal cell growth.

In the present application, the term "colon cancer" generally refers to a tumor that occurs in or originates from the digestive tract at the colon site. For example, the colon cancer can occur in or originate from the junction of the rectum with the sigmoid colon. For example, the colon cancer can be a cancer that occurs in or originates from the digestive tract at the colon site.

In the present application, the term "direct linkage" can be contrasted with the term "indirect linkage", which generally means direct connection. For example, the direct linkage can be a situation where the substances are linked directly without a spacer. The spacer can be a linker.

For example, the linker can be a peptide linker. The term "indirect linkage" generally refers to a situation where the substances are not linked directly. For example, the indirect linkage can be a situation where the substances are linked through a spacer. For example, in the isolated antigen-binding proteins of the present application, the C-terminus of the L-FR1 and the N-terminus of the LCDR1 can be linked directly or indirectly.

In the present application, the term "specific binding" or "specific" generally refers to a measurable and reproducible interaction, such as the binding between a target and an antibody, which can determine the presence of the target in the presence of a heterogeneous population of molecules, including biomolecules. For example, an antibody that specifically binds to a target (which can be an epitope) can be an antibody that binds to that target with greater affinity, avidity, more readily, and/or for a greater duration than it binds to other targets. In some embodiments, the antibody specifically binds to epitopes on a protein that are conserved among proteins of different species. In some embodiments, specific binding can include, but is not required to be exclusively binding.

In the present application, the term "subject" generally refers to a human or non-human animal, including, but not limited to, a cat, a dog, a horse, a pig, a cow, a sheep, rabbit, a mouse, a rat, or a monkey.

In the present application, the term "tumor" generally refers to any new pathological tissue hyperplasia. Tumor cells can spread to other parts of the body locally or through the blood stream and lymphatic system. In the present application, the tumor may include a benign tumor and a malignant tumor. In the present application, the tumor may include a solid tumor and/or a hematological tumor. In the present application, the tumors may include a caner. In the present application, examples of the tumor include, but are not limited to, colon cancer.

In the present application, reference to a protein, polypeptide and/or amino acid sequence is also to be understood as encompassing at least the following ranges: a variant or homologue having the same or similar function as said protein or polypeptide.

In the present application, the variant can be, for example, a protein or polypeptide having one or more amino acids have been substituted, deleted or added in the amino acid sequence of the protein and/or the polypeptide (e.g., an antibody or fragment thereof that specifically binds to Siglec). For example, the functional variant may comprise a protein or polypeptide that has been altered by at least 1, e.g., 1-30, 1-20 or 1-10, further e.g., 1, 2, 3, 4 or 5 amino acid substitution, deletion and/or insertion. The functional variant can substantially retain the biological properties of the protein or the polypeptide prior to alteration (e.g., substitution, deletion, or addition). For example, the functional variant can retain at least 60%, 70%, 80%, 90%, or 100% of the biological activity (e.g., antigen-binding ability) of the protein or the polypeptide prior to alteration. For example, the substitution can be a conservative substitution. For example, the variant can also be a polypeptide including a functionally active fragment thereof, not limited to a polypeptide including a functionally active fragment of the protein resulting from processing and/or modification occurring in a cell.

In the present application, the homologue can be a protein or polypeptide having at least about 85% (e.g., having at least about 85%, about 90%, about 91%, about 92%, about 93%, about 94%, about 95%, about 96%, about 97%, about 98%, about 99% or more) sequence homology to the amino acid sequence of the protein and/or the polypeptide (e.g., an antibody or fragment thereof that specifically binds to Siglec).

In the present application, the homology generally refers to similarity, similarity or association between two or more sequences. The "percentage of sequence homology" can be calculated by comparing the two sequences to be aligned over a comparison window, determining the number of positions at which the identical nucleic acid bases (e.g., A, T, C, G, I) or the identical amino acid residues (e.g., A la, Pro, Ser, Thr, Gly, Val, Leu, Ile, Phe, Tyr, Trp, Lys, Arg, His, Asp, Glu, Asn, Gln, Cys, and Met) occurs in the two sequences to yield the number of matched positions, dividing the number of matched positions by the total number of positions in the comparison window (i.e., the window size), and multiplying the result by 100 to yield the percentage of sequence homology. Alignment to determine the percentage of sequence homology can be accomplished in a variety of ways known in the art, for example, using publicly available computer software such as BLAST, BLAST-2, ALIGN, or Megalign (DNASTAR) software. Those skilled in the art can determine suitable parameters for the aligning sequences, including any algorithms required to achieve maximum alignment over the full length of the sequences being compared or over a region of the target sequence. The homology can also be determined by the following methods: FASTA and BLAST. The FASTA algorithm is described in W. R. Pearson and D. J. Lipman "Improved Tools for Biological Sequence Comparison", Proc. Natl. Acad. Sci., 85: 2444-2448, 1988; and D. J. Lipman and W. R. Pearson "Rapid and Sensitive Protein Similarity Searches", Science, 227:1435-1441,1989. The BLAST algorithm is described in S. Altschul, W. Gish, W. Miller, E. W. Myers and D. Lipman, A Basic Local Alignment Search Tool, J. Mol. Biol. 215: 403-410, 1990.

In the present application, the term "include or comprise" or other form of the term refers to the meaning of comprising, inclusive, containing, or encompassing. In some cases, it also represents the meanings of "is", and "consist of'.

In the present application, the term "about" generally refers to a range from 0.5% to 10% above or below the specified value, for example, a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### Detailed Description of The Invention

In one aspect, the present application provides an isolated antigen-binding protein.

The CDRs of an antibody, also known as the complementarity determining regions, are a portion of the variable region. The amino acid residues of this region can be contacted with an antigen or antigenic epitope. The antibody CDR can be identified by a variety of coding systems, which are known in the art, see, for example, www.bioinf.org.uk/abs/index.html#kabatnum. One skilled in the art can determine the CDR using different coding systems based on the sequence and structure of an antibody. There may be differences in the CDR with different coding systems. In the present application, the CDR covers the CDR sequence obtained by dividing according to any CDR dividing mode; the variants thereof are also contemplated, which comprises the amino acid sequence of the CDR with one or more amino acids substitution, deletion, and/or addition, for example, 1-30, 1-20 or 1-10, further for example, 1, 2, 3, 4, 5, 6, 7, 8 or 9 amino acid substitutions, deletions and/or insertions; the homologues thereof are also contemplated, which can be amino acid sequences having at least about 85% (e.g., having at least about 85%, about 90%, about 91%, about 92%, about 93%, about 94%, about 95%, about 96%, about 97%, about 98%, about 99% or more) sequence homology to the amino acid sequence of the CDR. In some embodiments, the CDR is determined by a Kabat numbering scheme. In some embodiments, the CDR is determined by a Chothia numbering scheme.

In one aspect, the present application provides an isolated antigen-binding protein comprising HCDR3 comprising an amino acid sequence as set forth in AIGSSWYSDAFDL (SEQ ID NO: 1). For example, the isolated antigen-binding protein of the present application can have Siglec-15 binding ability. For example, the isolated antigen-binding protein may comprise HCDR3 in Ab0, Ab2, Ab3, Ab5, Ab6, Ab7, Ab9, Ab13, or Ab15 of the present application. For example, the isolated antigen-binding protein may comprise HCDR3 in the heavy chain variable region of Ab0, Ab2, Ab3, Ab5, Ab6, Ab7, Ab9, Ab13, or Ab15 of the present application. For example, the CDR is determined according to the Chothia rule.

In the present application, the isolated antigen-binding protein can further comprise HCDR2. For example, the HCDR2 may comprise a sequence as set forth in X₁GGGX₂X₃, wherein X₁, X₂, and X₃ can each independently be selected from any amino acid, such as a natural amino acid. For example, the HCDR2 may comprise a sequence as set forth in X₁GGGX₂X₃ (SEQ ID NO: 33), wherein X₁ can be S, T, W or Y, X₂ can be E, G or V, and X₃ can be S or Y In some cases, compared to the amino acid sequence as set forth in SEQ ID NO: 2, the HCDR2 may comprise at least an amino acid substitution at a position selected from the group consisting of amino acid substitutions at X₁, X₂ and X₃. For example, the HCDR2 may comprise an amino acid sequence as set forth in SEQ ID NO: 33. For example, the isolated antigen-binding protein of the present application can have Siglec-15 binding ability.

For example, the HCDR2 can comprises an amino acid sequence as set forth in SGGGGS (SEQ ID NO: 2), TGGGES (SEQ ID NO: 3), YGGGGS (SEQ ID NO: 4), WGGGGS (SEQ ID NO: 5), or SGGGVY (SEQ ID NO: 6). For example, the isolated antigen-binding protein of the present application can have Siglec-15 binding ability. For example, the isolated antigen-binding protein may comprise HCDR2 in Ab0, Ab2, Ab3, Ab5, Ab6, Ab7, Ab9, Ab13, or Ab15 of the present application. For example, the isolated antigen-binding protein may comprise HCDR2 in the heavy chain variable region of Ab0, Ab2, Ab3, Ab5, Ab6, Ab7, Ab9, Ab13, or Ab15 of the present application. For example, the CDR is determined according to the Chothia rule.

In the present application, the isolated antigen-binding protein can further comprise HCDR1. For example, the HCDR1 may comprise a sequence as set forth in GFTX₄SSY, wherein X₄ can be selected from any amino acid, such as natural amino acids. For example, the HCDR1 may comprise a sequence as set forth in GFTX₄SSY (SEQ ID NO: 34), wherein X₄ can be F or S. In some cases, compared to the amino acid sequence as set forth in SEQ ID NO: 7, the HCDR1 may comprise at least an amino acid substitution at X₄. For example, the HCDR1 may comprise an amino acid sequence as set forth in SEQ ID NO: 34. For example, the isolated antigen-binding protein of the present application can have Siglec-15 binding ability.

For example, the HCDR1 may comprise an amino acid sequence as set forth in GFTFSSY (SEQ ID NO: 7) or GFTSSSY (SEQ ID NO: 8). For example, the isolated antigen-binding protein of the present application can have Siglec-15 binding ability. For example, the isolated antigen-binding protein may comprise HCDR1 in Ab0, Ab2, Ab3, Ab5, Ab6, Ab7, Ab9, Ab13, or Ab 15 of the present application. For example, the isolated antigen-binding protein may comprise HCDR1 in the heavy chain variable region of Ab0, Ab2, Ab3, Ab5, Ab6, Ab7, Ab9, Ab13, or Ab15 of the present application. For example, the CDR is determined according to the Chothia rule.

In the present application, the isolated antigen-binding protein may comprise HCDR3, HCDR2, and HCDR1. For example, HCDR3 may comprise a sequence as set forth in SEQ ID NO: 1, HCDR2 may comprise a sequence as set forth in X₁GGGX₂X₃, wherein X₁, X₂, and X₃ can each independently be selected from any amino acid, such as a natural amino acid, and HCDR1 may comprise a sequence as set forth in GFTX₄SSY, wherein X₄ can be selected from any amino acid, such as a natural amino acid. For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 33; and the HCDR1 of the isolated antigen-binding protein may comprise an amino acid sequence as set forth in SEQ ID NO: 34. For example, the isolated antigen-binding protein of the present application can have Siglec-15 binding ability.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 2; and the HCDR1 of the isolated antigen-binding protein may comprise an amino acid sequence as set forth in SEQ ID NO: 7. For example, the isolated antigen-binding protein of the present application can have Siglec-15 binding ability. For example, the isolated antigen-binding protein may comprise HCDR1, HCDR2, and HCDR3 of Ab0 or Ab9 of the present application. For example, the isolated antigen-binding protein may comprise HCDR1, HCDR2, and HCDR3 in the heavy chain variable region of Ab0 or Ab9 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 2; and the HCDR1 of the isolated antigen-binding protein may comprise an amino acid sequence as set forth in SEQ ID NO: 8. For example, the isolated antigen-binding protein of the present application can have Siglec-15 binding ability. For example, the isolated antigen-binding protein may comprise HCDR1, HCDR2, and HCDR3 of Ab2 of the present application. For example, the isolated antigen-binding protein may comprise HCDR1, HCDR2, and HCDR3 in the heavy chain variable region of Ab2 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 3; and the HCDR1 of the isolated antigen-binding protein may comprise an amino acid sequence as set forth in SEQ ID NO: 7. For example, the isolated antigen-binding protein of the present application can have Siglec-15 binding ability. For example, the isolated antigen-binding protein may comprise HCDR1, HCDR2, and HCDR3 of Ab3, Ab13, or Ab15 of the present application. For example, the isolated antigen-binding protein may comprise HCDR1, HCDR2, and HCDR3 in the heavy chain variable region of Ab3, Ab13, or Ab15 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 4; and the HCDR1 of the isolated antigen-binding protein may comprise an amino acid sequence as set forth in SEQ ID NO: 7. For example, the isolated antigen-binding protein of the present application can have Siglec-15 binding ability. For example, the isolated antigen-binding protein may comprise HCDR1, HCDR2, and HCDR3 of Ab5 of the present application. For example, the isolated antigen-binding protein may comprise HCDR1, HCDR2, and HCDR3 in the heavy chain variable region of Ab5 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 5; and the HCDR1 of the isolated antigen-binding protein may comprise an amino acid sequence as set forth in SEQ ID NO: 7. For example, the isolated antigen-binding protein of the present application can have Siglec-15 binding ability. For example, the isolated antigen-binding protein may comprise HCDR1, HCDR2, and HCDR3 of Ab6 of the present application. For example, the isolated antigen-binding protein may comprise HCDR1, HCDR2, and HCDR3 in the heavy chain variable region of Ab6 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 6; and the HCDR1 of the isolated antigen-binding protein may comprise an amino acid sequence as set forth in SEQ ID NO: 7. For example, the isolated antigen-binding protein of the present application can have Siglec-15 binding ability. For example, the isolated antigen-binding protein may comprise HCDR1, HCDR2, and HCDR3 of Ab7 of the present application. For example, the isolated antigen-binding protein may comprise HCDR1, HCDR2, and HCDR3 in the heavy chain variable region of Ab7 of the present application. For example, the CDR is determined according to the Chothia rule.

In the present application, the isolated antigen-binding protein may comprise LCDR3, which may comprise an amino acid sequence as set forth in QQSYSIPYT (SEQ ID NO: 9). For example, the isolated antigen-binding protein of the present application can have Siglec-15 binding ability. For example, the isolated antigen-binding protein may comprise LCDR3 in Ab0, Ab2, Ab3, Ab5, Ab6, Ab7, Ab9, Ab13, or Ab15 of the present application. For example, the isolated antigen-binding protein may comprise LCDR3 in the light chain variable region of Ab0, Ab2, Ab3, Ab5, Ab6, Ab7, Ab9, Ab13, or Ab15 of the present application. For example, the CDR is determined according to the Chothia rule.

In the present application, the isolated antigen-binding protein can further comprise LCDR2. For example, the LCDR2 may comprise a sequence as set forth in VASX₅X₆X₇X₈, wherein X₅, X₆, X₇, and X₈ can each independently be selected from any amino acid, such as a natural amino acid. For example, the LCDR2 may comprise a sequence as set forth in VASX₅X₆X₇X₈ (SEQ ID NO: 35), wherein X₅ can be F or S, X₆ can be E, I or L, X₇ can be H or Q, and X₈ can be R or S. In some cases, compared to the amino acid sequence as set forth in SEQ ID NO: 10, the LCDR2 may comprise at least an amino acid substitution at a position selected from the group consisting of amino acid substitutions at X₅, X₆, X₇, and X₈. For example, the LCDR2 may comprise an amino acid sequence as set forth in SEQ ID NO: 35. For example, the isolated antigen-binding protein of the present application can have Siglec-15 binding ability.

For example, the LCDR2 may comprise an amino acid sequence as set forth in VASSLQS (SEQ ID NO: 10), VASFEHR (SEQ ID NO: 11), or VASYIHS (SEQ ID NO: 12). For example, the isolated antigen-binding protein of the present application can have Siglec-15 binding ability. For example, the isolated antigen-binding protein may comprise LCDR2 in Ab0, Ab2, Ab3, Ab5, Ab6, Ab7, Ab9, Ab13, or Ab15 of the present application. For example, the isolated antigen-binding protein may comprise LCDR2 in the light chain variable region of Ab0, Ab2, Ab3, Ab5, Ab6, Ab7, Ab9, Ab13, or Ab15 of the present application. For example, the CDR is determined according to the Chothia rule.

In the present application, the isolated antigen-binding protein can further comprise LCDR1. For example, the LCDR1 may comprise a sequence as set forth in RASQDISX₉WLA, wherein X₉ can be selected from any amino acid, such as natural amino acids. For example, the LCDR1 may comprise a sequence as set forth in RASQDISX₉WLA (SEQ ID NO: 36), wherein X₉ can be D or S. In some cases, compared to the amino acid sequence as set forth in SEQ ID NO: 13, the LCDR1 may comprise at least an amino acid substitution at X₉. For example, the LCDR1 may comprise an amino acid sequence as set forth in SEQ ID NO: 36. For example, the isolated antigen-binding protein of the present application can have Siglec-15 binding ability.

For example, the LCDR1 may comprise an amino acid sequence as set forth in RASQDISSWLA (SEQ ID NO: 13) or RASQDISDWLA (SEQ ID NO: 14). For example, the isolated antigen-binding protein of the present application can have Siglec-15 binding ability. For example, the isolated antigen-binding protein may comprise LCDR1 in Ab0, Ab2, Ab3, Ab5, Ab6, Ab7, Ab9, Ab13, or Ab15 of the present application. For example, the isolated antigen-binding protein may comprise LCDR1 in the light chain variable region of Ab0, Ab2, Ab3, Ab5, Ab6, Ab7, Ab9, Ab13, or Ab15 of the present application. For example, the CDR is determined according to the Chothia rule.

In the present application, the isolated antigen-binding protein may comprise LCDR3, LCDR2, and LCDR1. For example, LCDR3 may comprise a sequence as set forth in SEQ ID NO: 9, LCDR2 may comprise a sequence as set forth in VASX₅X₆X₇X₈, wherein X₅, X₆, X₇, and X₈ can each independently be selected from any amino acid, such as a natural amino acid, and LCDR1 may comprise a sequence as set forth in RASQDISX₉WLA, wherein X₉ can be selected from any amino acid, such as a natural amino acid. For example, the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 35; and the LCDR1 of the isolated antigen-binding protein may comprise an amino acid sequence as set forth in SEQ ID NO: 36. For example, the isolated antigen-binding protein of the present application can have Siglec-15 binding ability.

For example, the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise LCDR1, LCDR2, and LCDR3 of Ab0, Ab2, Ab3, Ab5, or Ab7 of the present application. For example, the isolated antigen-binding protein may comprise LCDR1, LCDR2, and LCDR3 in the light chain variable region of Ab0, Ab2, Ab3, Ab5, or Ab7 of the present application. For example, the CDR is determined according to the Chothia rule.

For example, the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 14. For example, the isolated antigen-binding protein of the present application can have Siglec-15 binding ability. For example, the isolated antigen-binding protein may comprise LCDR1, LCDR2, and LCDR3 of Ab9 of the present application. For example, the isolated antigen-binding protein may comprise LCDR1, LCDR2, and LCDR3 in the light chain variable region of Ab9 of the present application. For example, the CDR is determined according to the Chothia rule.

For example, the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 11; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise LCDR1, LCDR2, and LCDR3 in Ab13 of the present application. For example, the isolated antigen-binding protein may comprise LCDR1, LCDR2, and LCDR3 in the light chain variable region in Ab13 of the present application. For example, the CDR is determined according to the Chothia rule.

For example, the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 12; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise LCDR1, LCDR2, and LCDR3 in Ab15 of the present application. For example, the isolated antigen-binding protein may comprise LCDR1, LCDR2, and LCDR3 in the light chain variable region of Ab15 of the present application. For example, the CDR is determined according to the Chothia rule.

In the present application, the isolated antigen-binding protein may comprise HCDR3, HCDR2, HCDR1, LCDR3, LCDR2, and LCDR1. For example, HCDR3 may comprise a sequence as set forth in SEQ ID NO: 1; HCDR2 may comprise a sequence as set forth in X₁GGGX₂X₃, wherein X₁, X₂, and X₃ can each independently be selected from any amino acid, such as a natural amino acid; and HCDR1 may comprise a sequence as set forth in GFTX₄SSY, wherein X₄ can be selected from any amino acid, such as a natural amino acid; LCDR3 may comprise a sequence as set forth in SEQ ID NO: 9; LCDR2 may comprise a sequence as set forth in VASX₅X₆X₇X₈, wherein X₅, X₆, X₇, and X₈ can each independently be selected from any amino acid, such as a natural amino acid; and LCDR1 may comprise a sequence as set forth in RASQDISX₉WLA, wherein X₉ can be selected from any amino acid, such as a natural amino acid. For example, HCDR3 may comprise a sequence as set forth in SEQ ID NO: 1; HCDR2 may comprise a sequence as set forth in X₁GGGX₂X₃ (SEQ ID NO: 33), wherein X₁ can be S, T, W or Y, X₂ can be E, G or V, and X₃ can be S or Y; and HCDR1 may comprise a sequence as set forth in GFTX₄SSY (SEQ ID NO: 34), wherein X₄ can be F or S; LCDR3 may comprise a sequence as set forth in SEQ ID NO: 9; LCDR2 may comprise a sequence as set forth in VASX₅X₆X₇X₈ (SEQ ID NO: 35), wherein X₅ can be F, S, or Y, X₆ can be E, I, or L, X₇ can be H or Q, and X₈ can be R or S; and LCDR3 may comprise a sequence as set forth in RASQDISX₉WLA (SEQ ID NO: 36), wherein X₉ can be D or S. In some cases, compared to the amino acid sequence as set forth in SEQ ID NO: 2, the HCDR2 may comprise at least an amino acid substitution at a position selected from the group consisting of amino acid substitutions at X₁, X₂ and X₃; compared to the amino acid sequence as set forth in SEQ ID NO: 7, the HCDR1 may comprise at least an amino acid substitution at X₄; compared to the amino acid sequence as set forth in SEQ ID NO: 10, the LCDR2 may comprise at least an amino acid substitution at a position selected from the group consisting of amino acid substitutions at X₅, X₆, X₇, and X₈; and compared to the amino acid sequence as set forth in SEQ ID NO: 13, the LCDR1 may comprise at least an amino acid substitution at X₉. For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 33; and the HCDR1 of the isolated antigen-binding protein may comprise an amino acid sequence as set forth in SEQ ID NO: 34; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 35; and the LCDR1 of the isolated antigen-binding protein may comprise an amino acid sequence as set forth in SEQ ID NO: 36. For example, the isolated antigen-binding protein of the present application can have Siglec-15 binding ability.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 2; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab0 of the present application. For example, the isolated antigen-binding protein may comprise HCDR3, HCDR2, HCDR1 in the heavy chain variable region, and LCDR3, LCDR2, and LCDR1 in the light chain variable region of Ab0 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 2; and the HCDR1 of the isolated antigen-binding protein may comprise an amino acid sequence as set forth in SEQ ID NO: 8; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab2 of the present application. For example, the isolated antigen-binding protein may comprise HCDR3, HCDR2, HCDR1 in the heavy chain variable region, and LCDR3, LCDR2, and LCDR1 in the light chain variable region of Ab2 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 3; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab3 of the present application. For example, the isolated antigen-binding protein may comprise HCDR3, HCDR2, HCDR1 in the heavy chain variable region, LCDR3, LCDR2, and LCDR1 in the light chain variable region of Ab3 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 4; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab5 of the present application. For example, the isolated antigen-binding protein may comprise HCDR3, HCDR2, HCDR1 in the heavy chain variable region, and LCDR3, LCDR2, and LCDR1 in the light chain variable region of Ab5 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 5; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab6 of the present application. For example, the isolated antigen-binding protein may comprise HCDR3, HCDR2, HCDR1 in the heavy chain variable region, and LCDR3, LCDR2, and LCDR1 in the light chain variable region of Ab6 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 6; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 inAb7 of the present application. For example, the isolated antigen-binding protein may comprise HCDR3, HCDR2, HCDR1 in the heavy chain variable region, and LCDR3, LCDR2, and LCDR1 in the light chain variable region of Ab7 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 2; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 10; and the LCDR1 of the isolated antigen-binding protein may comprise an amino acid sequence as set forth in SEQ ID NO: 14. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab9 of the present application. For example, the isolated antigen-binding protein may comprise HCDR3, HCDR2, HCDR1 in the heavy chain variable region, and LCDR3, LCDR2, and LCDR1 in the light chain variable region of Ab9 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 3; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 11; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab13 of the present application. For example, the isolated antigen-binding protein may comprise HCDR3, HCDR2, HCDR1 in the heavy chain variable region, and LCDR3, LCDR2, and LCDR1 in the light chain variable region of Ab13 of the present application. For example, the CDR is determined according to the Chothia rule.

For example, the HCDR3 of the isolated antigen-binding protein may comprise an amino acid sequence as set forth in SEQ ID NO: 1; the HCDR2 of the isolated antigen-binding protein may comprise an amino acid sequence as set forth in SEQ ID NO: 3; and the HCDR1 of the isolated antigen-binding protein may comprise an amino acid sequence as set forth in SEQ ID NO: 7; the LCDR3 of the isolated antigen-binding protein may comprise an amino acid sequence as set forth in SEQ ID NO: 9; the LCDR2 of the isolated antigen-binding protein may comprise an amino acid sequence as set forth in SEQ ID NO: 12; and the LCDR1 of the isolated antigen-binding protein may comprise an amino acid sequence as set forth in SEQ ID NO: 13. For example, the isolated antigen-binding protein of the present application can have Siglec-15 binding ability. For example, the isolated antigen-binding protein may comprise HCDR3, HCDR2, HCDR1, LCDR3, LCDR2, and LCDR1 in Ab15 of the present application. For example, the isolated antigen-binding protein may comprise HCDR3, HCDR2, HCDR1 in the heavy chain variable region, and LCDR3, LCDR2, and LCDR1 in the light chain variable region of Ab15 of the present application. For example, the CDR is determined according to the Chothia rule.

In the present application, the isolated antigen-binding protein may comprise H-FR1, wherein the C-terminus of the HCDR1 can be directly or indirectly linked to the N-terminus of the HCDR1, and the H-FR1 may comprise an amino acid sequence as set forth in SEQ ID NO: 15.

In the present application, the isolated antigen-binding protein comprises H-FR2, wherein the H-FR2 is located between the HCDR1 and the HCDR2, and the H-FR2 may comprise an amino acid sequence as set forth in SEQ ID NO: 16.

In the present application, the isolated antigen-binding protein may comprise H-FR3, wherein the H-FR3 is located between the HCDR2 and the HCDR3, and the H-FR3 may comprise an amino acid sequence as set forth in SEQ ID NO: 17.

In the present application, the isolated antigen-binding protein may comprise H-FR4, wherein the N-terminus of the H-FR4 can be linked to the C-terminus of the HCDR3, and the H-FR4 may comprise an amino acid sequence as set forth in SEQ ID NO: 18.

In the present application, the antigen-binding protein may comprise H-FR1, H-FR2, H-FR3, and H-FR4. For example, the H-FR1, H-FR2, H-FR3, and H-FR4 of the isolated antigen-binding protein may comprise amino acid sequences as set forth in SEQ ID NO: 15, SEQ ID NO: 16, SEQ ID NO: 17, and SEQ ID NO: 18, respectively.

In the present application, the isolated antigen-binding protein may comprise L-FR1, wherein the C-terminus of the L-FR1 can be directly or indirectly linked to the N-terminus of the LCDR1, and the L-FR1 may comprise an amino acid sequence as set forth in SEQ ID NO: 19.

In the present application, the isolated antigen-binding protein may comprise L-FR2, wherein the L-FR2 can be located between the LCDR1 and the LCDR2, and L-FR2 may comprise an amino acid sequence as set forth in SEQ ID NO: 20.

In the present application, the isolated antigen-binding protein comprises L-FR3, wherein the L-FR3 is located between the LCDR2 and the LCDR3, and the L-FR3 may comprise an amino acid sequence as set forth in SEQ ID NO: 21.

In the present application, the isolated antigen-binding protein may comprise L-FR4, wherein the N-terminus of the L-FR4 can be directly or indirectly linked to the C-terminus of the LCDR3, and the L-FR4 may comprise an amino acid sequence as set forth in SEQ ID NO: 22.

In the present application, the isolated antigen-binding protein may comprise L-FR1, L-FR2, L-FR3, and L-FR4. For example, the L-FR1, L-FR2, L-FR3, and L-FR4 of the isolated antigen-binding protein may comprise amino acid sequences as set forth in SEQ ID NO: 19, SEQ ID NO: 20, SEQ ID NO: 21, and SEQ ID NO: 22, respectively.

In the present application, the isolated antigen-binding protein may comprise VH, which may comprise an amino acid sequence as set forth in SEQ ID NO: 37. For example, the VH may comprise an amino acid sequence as set forth in EVQLLESGGGLVQPGGSLRLSCAASGFTX₄SSYAMSWVRQAPGKGLEWVSTIX₁GGGX₂X ₃TYYADSVKGRFTISRDNSKNTLYLQMNSLRAEDTAVYYCAKAIGSSWYSDAFDLWGQG TMVTVSS (SEQ ID NO: 37), wherein X₁ can be S, T, W, or Y, X₂ can be E, G, or V, X₃ can be S or Y, and X₄ can be F or S. In some cases, compared to the amino acid sequence as set forth in SEQ ID NO: 23, the VH may comprise at least an amino acid substitution at a position selected from the group consisting of amino acid substitutions at X₁, X₂, X₃, and X₄. For example, the isolated antigen-binding protein of the present application can have Siglec-15 binding ability.

In the present application, the antigen-binding protein may comprise VH, which may comprise an amino acid sequence as set forth in SEQ ID NO: 23, SEQ ID NO: 24, SEQ ID NO: 25, SEQ ID NO: 26, SEQ ID NO: 27, or SEQ ID NO: 28. For example, the isolated antigen-binding protein of the present application can have Siglec-15 binding ability. For example, the isolated antigen-binding protein may comprise a heavy chain variable region in Ab0, Ab2, Ab3, Ab5, Ab6, Ab7, Ab9, Ab13, or Ab15 of the present application.

In the present application, the isolated antigen-binding protein may comprise VL, which may comprise an amino acid sequence as set forth in SEQ ID NO: 38. For example, the VL may comprise an amino acid sequence as set forth in DIQMTQSPSSVSASVGDRVTITCRASQDISX₉WLAWYQQKPGKAPKLLIYVASX₅X₆X₇X₈G VPSRFSGSGSGTDFTLTISSLQPEDFATYYCQQSYSIPYTFGQGTKLEIK (SEQ ID NO: 38), wherein X₅ can be F, S, or Y, X₆ can be E, I, or L, X₇ can be H or Q, and X₈ can be R or S, and X₉ can be D or S. In some cases, compared to the amino acid sequence as set forth in SEQ ID NO: 29, the VH may comprise at least an amino acid substitution at a position selected from the group consisting of amino acid substitutions at X₅, X₆, X₇, X₈, and X₉. For example, the isolated antigen-binding protein of the present application can have Siglec-15 binding ability.

In the present application, the antigen-binding protein may comprise VL which may comprise an amino acid sequence as set forth in SEQ ID NO: 29, SEQ ID NO: 30, SEQ ID NO: 31, or SEQ ID NO: 32. For example, the isolated antigen-binding protein of the present application can have Siglec-15 binding ability. For example, the isolated antigen-binding protein may comprise a light chain variable region in Ab0, Ab2, Ab3, Ab5, Ab6, Ab7, Ab9, Ab 13, or Ab 15 of the present application.

In the present application, the isolated antigen-binding protein may comprise the VH and VL. For example, the VH may comprise an amino acid sequence as set forth in SEQ ID NO: 37 and the VL may comprise an amino acid sequence as set forth in SEQ ID NO: 38. For example, the isolated antigen-binding protein of the present application can have Siglec-15 binding ability.

For example, the VH may comprise an amino acid sequence as set forth in SEQ ID NO: 23 and the VL may comprise an amino acid sequence as set forth in SEQ ID NO: 29. For example, the isolated antigen-binding protein of the present application can have Siglec-15 binding ability. For example, the isolated antigen-binding protein may comprise the VH and VL in Ab0 of the present application.

For example, the VH may comprise an amino acid sequence as set forth in SEQ ID NO: 24 and the VL may comprise an amino acid sequence as set forth in SEQ ID NO: 29. For example, the isolated antigen-binding protein of the present application can have Siglec-15 binding ability. For example, the isolated antigen-binding protein may comprise the VH and VL in Ab2 of the present application.

For example, the VH may comprise an amino acid sequence as set forth in SEQ ID NO: 25 and the VL may comprise an amino acid sequence as set forth in SEQ ID NO: 29. For example, the isolated antigen-binding protein of the present application can have Siglec-15 binding ability. For example, the isolated antigen-binding protein may comprise the VH and VL in Ab3 of the present application.

For example, the VH may comprise an amino acid sequence as set forth in SEQ ID NO: 26 and the VL may comprise an amino acid sequence as set forth in SEQ ID NO: 29. For example, the isolated antigen-binding protein of the present application can have Siglec-15 binding ability. For example, the isolated antigen-binding protein may comprise the VH and VL in Ab5 of the present application.

For example, the VH may comprise an amino acid sequence as set forth in SEQ ID NO: 27 and the VL may comprise an amino acid sequence as set forth in SEQ ID NO: 29. For example, the isolated antigen-binding protein of the present application can have Siglec-15 binding ability. For example, the isolated antigen-binding protein may comprise the VH and VL in Ab6 of the present application.

For example, the VH may comprise an amino acid sequence as set forth in SEQ ID NO: 28 and the VL may comprise an amino acid sequence as set forth in SEQ ID NO: 29. For example, the isolated antigen-binding protein of the present application can have Siglec-15 binding ability. For example, the isolated antigen-binding protein may comprise the VH and VL in Ab7 of the present application.

For example, the VH may comprise an amino acid sequence as set forth in SEQ ID NO: 23 and the VL may comprise an amino acid sequence as set forth in SEQ ID NO: 30. For example, the isolated antigen-binding protein of the present application can have Siglec-15 binding ability. For example, the isolated antigen-binding protein may comprise the VH and VL in Ab9 of the present application.

For example, the VH may comprise an amino acid sequence as set forth in SEQ ID NO: 25 and the VL may comprise an amino acid sequence as set forth in SEQ ID NO: 31. For example, the isolated antigen-binding protein of the present application can have Siglec-15 binding ability. For example, the isolated antigen-binding protein may comprise the VH and VL in Ab 13 of the present application.

For example, the VH may comprise an amino acid sequence as set forth in SEQ ID NO: 25 and the VL may comprise an amino acid sequence as set forth in SEQ ID NO: 32. For example, the isolated antigen-binding protein of the present application can have Siglec-15 binding ability. For example, the isolated antigen-binding protein may comprise the VH and VL in Ab 15 of the present application.

In the present application, the isolated antigen-binding protein may comprise at least one CDR in any of the VHs of the present application. In the present application, the isolated antigen-binding protein may comprise at least one CDR in any of the VLs of the present application. The CDR can be determined according to any division scheme, such as CCG, Kabat, Chothia, IMGT, comprehensively considering Kabat/Chothia, etc.

In the present application, the isolated antigen-binding protein may comprise HCDR1, HCDR2, and HCDR3 in VH of the present application, wherein the VH may comprise an amino acid sequence as set forth in SEQ ID NO: 37. For example, the isolated antigen-binding protein of the present application can have Siglec-15 binding ability.

In the present application, the antigen-binding protein may comprise HCDR1, HCDR2, and HCDR3 in VH of the present application, wherein the VH may comprise an amino acid sequence as set forth in SEQ ID NO: 23, SEQ ID NO: 24, SEQ ID NO: 25, SEQ ID NO: 26, SEQ ID NO: 27, or SEQ ID NO: 28. For example, the isolated antigen-binding protein of the present application can have Siglec-15 binding ability.

In the present application, the isolated antigen-binding protein may comprise LCDR1, LCDR2, and LCDR3 in VL of the present application, wherein the VL may comprise an amino acid sequence as set forth in SEQ ID NO: 38. For example, the isolated antigen-binding protein of the present application can have Siglec-15 binding ability.

In the present application, the antigen-binding protein may comprise LCDR1, LCDR2, and LCDR3 in VL of the present application, wherein the VL may comprise an amino acid sequence as set forth in SEQ ID NO: 29, SEQ ID NO: 30, SEQ ID NO: 31, or SEQ ID NO: 32. For example, the isolated antigen-binding protein of the present application can have Siglec-15 binding ability.

In the present application, the isolated antigen-binding protein may include an antibody heavy chain constant region. The antibody heavy chain constant region can be derived from a human IgG, IgA, IgD, IgE and/or IgM heavy chain constant region. The antibody heavy chain constant region can be derived from a human IgG heavy chain constant region. In some embodiments, the isolated antigen-binding protein may include an antibody heavy chain constant region, and the antibody heavy chain constant region can be derived from a human IgG1, IgG2, IgG3, and/or IgG4 heavy chain constant region. In some embodiments, the isolated antigen-binding protein may include an antibody heavy chain constant region, and the antibody heavy chain constant region can be derived from a human IgG1 heavy chain constant region.

In the present application, the isolated antigen-binding protein may include an antibody light chain constant region. The antibody light chain constant region can be derived from a human Igκ constant region.

In the present application, the isolated antigen-binding protein may comprise an antibody or antigen-binding fragment thereof.

In some embodiments, the antigen-binding fragment may include Fab, Fab', a Fv fragment, F(ab')₂, F(ab)₂, scFv, di-scFv, VHH, and/or dAb.

In some embodiments, the antibody may include a monoclonal antibody. In some embodiments, the antibodies may include a murine antibody, a chimeric antibody, a humanized antibody and/or a fully human antibody.

For example, the VH of the chimeric antibody may comprise an amino acid sequence as set forth in SEQ ID NO: 23 and the VL of the chimeric antibody may comprise an amino acid sequence as set forth in SEQ ID NO: 29.

In some embodiments, the VH of the humanized antibody may comprise an amino acid sequence as set forth in SEQ ID NO: 37 and the VL of the humanized antibody may comprise an amino acid sequence as set forth in SEQ ID NO: 38. For example, the VH may comprise an amino acid sequence as set forth in SEQ ID NO: 23 and the VL may comprise an amino acid sequence as set forth in SEQ ID NO: 29. For example, the VH may comprise an amino acid sequence as set forth in SEQ ID NO: 24 and the VL may comprise an amino acid sequence as set forth in SEQ ID NO: 29. For example, the VH may comprise an amino acid sequence as set forth in SEQ ID NO: 25 and the VL may comprise an amino acid sequence as set forth in SEQ ID NO: 29. For example, the VH may comprise an amino acid sequence as set forth in SEQ ID NO: 26 and the VL may comprise an amino acid sequence as set forth in SEQ ID NO: 29. For example, the VH may comprise an amino acid sequence as set forth in SEQ ID NO: 27 and the VL may comprise an amino acid sequence as set forth in SEQ ID NO: 29. For example, the VH may comprise an amino acid sequence as set forth in SEQ ID NO: 28 and the VL may comprise an amino acid sequence as set forth in SEQ ID NO: 29. For example, the VH may comprise an amino acid sequence as set forth in SEQ ID NO: 23 and the VL may comprise an amino acid sequence as set forth in SEQ ID NO: 30. For example, the VH may comprise an amino acid sequence as set forth in SEQ ID NO: 25 and the VL may comprise an amino acid sequence as set forth in SEQ ID NO: 31. For example, the VH may comprise an amino acid sequence as set forth in SEQ ID NO: 25 and the VL may comprise an amino acid sequence as set forth in SEQ ID NO: 32.

In some embodiments, the antibody comprises a light chain constant region. For example, the light chain constant region can be a light chain constant region derived from a human antibody; for example, the light chain constant region may comprise an amino acid sequence as set forth in SEQ ID NO:40.

In some embodiments, the antibody comprises a heavy chain constant region. For example, the heavy chain constant region can be a heavy chain constant region derived from a human antibody; for example, the heavy chain constant region may comprise an amino acid sequence as set forth in SEQ ID NO:39.

In addition, it is noted that the isolated antigen-binding proteins of the present application may comprise heavy and/or light chain sequences with which one or more conservative sequence modifications thereto. By "conservative sequence modification" is meant amino acid modifications that do not significantly affect or alter the binding characteristics of the antibody. Such conservative modifications include amino acid substitutions, additions, and deletions. Modifications can be introduced into the isolated antigen-binding proteins of the present application by standard techniques known in the art, such as point mutations and PCR-mediated mutations. Conservative amino acid substitutions are those in which an amino acid residue is replaced with an amino acid residue having a similar side chain. Groups of amino acid residues having similar side chains are known in the art. These groups of amino acid residues include amino acids having basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). In some embodiments, one or more amino acid residues in the CDR of the isolated antigen-binding protein of the present application can be replaced with other amino acid residues of the same side chain group. Those skilled in the art will recognize that some conservative sequence modifications do not abolish antigen binding, see, for example, Brummell et al., (1993) Biochem 32: 1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem.32:6862-35; Adib-Conquy et al., (1998) Int. Immunol.10:341-6 and Beers et al., (2000) Clin. Can. Res. 6:2835-43.

The Siglec-15 antigen-binding proteins of the present application can be identified, screened, or characterized by a variety of assays known in the art.

For example, an antigen-binding protein or fusion protein of the present application can be tested for antigen-binding activity by known methods such as enzyme-linked immunosorbent assay (ELISA), immunoblot (e.g., Western blot), flow cytometry (e.g., FACS), immunohistochemistry, and immunofluorescence, etc.

In the present application, the isolated antigen-binding protein is capable of specifically binding to Siglec or a functionally active fragment thereof, such as Siglec-15 or a functionally active fragment thereof.

In some embodiments, Siglec-15 or a functionally active fragment thereof can be full-length Siglec-15 or a functionally active fragment thereof, or a fragment that exerts Siglec-15 functional activity, e.g., Siglec-15 functional activity can be the ability to activate a downstream pathway of Siglec-15. In some embodiments, Siglec-15 or a functionally active fragment thereof can be isolated Siglec-15 or a functionally active fragment thereof, or a mixture of various forms of Siglec-15 or a functionally active fragment thereof. For example, Siglec-15 or a functionally active fragment thereof can be human Siglec-15 or a functionally active fragment thereof, murine Siglec-15 or a functionally active fragment thereof or monkey Siglec-15 or a functionally active fragment thereof; for example, Siglec-15 or a functionally active fragment thereof can be Siglec-15 or a functionally active fragment thereof expressed on the surface of a cell; for example, Siglec-15 or a functionally active fragment thereof can be Siglec-15 or a functionally active fragment thereof bound to a solid phase medium.

In some embodiments, the isolated antigen-binding protein can be measured for affinity by a molecular interaction analyzer. For example, the isolated antigen-binding protein can bind to Siglec-15 protein with a K_{D} value of 1.56E-10 M or less. For example, the isolated antigen-binding protein can bind to Siglec-15 protein with a K_{D} value of 1.56E-10 M or less, 1.5E-10 M or less, 1.3E-10 M or less, 1.1E-10 M or less, 1.0E-10 M or less, 9.0E-11 M or less, 8.0E-11 M or less, 7.0E-11 M or less, 6.0E-11 M or less, 5.0E-11 M or less, 4.0E-11 M or less, 3.0E-11 M or less, 2.0E-11 M or less, 1.0E-11 M or less, 9.0E-12 M or less, 8.0E-12 M or less, 7.0E-12 M or less, 6.0E-12 M or less, or 5.0E-12 M or less.

In some embodiments, the isolated antigen-binding proteins of the present application can be expressed at higher amounts. For example, the isolated antigen-binding protein of the present application can be expressed in an amount of 50 mg/L or more. For example, the isolated antigen-binding protein of the present application can be expressed in an amount of 50 mg/L or more, 53 mg/L or more, 55 mg/L or more, 60 mg/L or more, 70 mg/L or more, 80 mg/L or more, 90 mg/L or more, 100 mg/L or more, 130 mg/L or more, 140 mg/L or more, 150 mg/L or more, 160 mg/L or more, 200 mg/L or more, 210 mg/L or more, 220 mg/L or more, or 230 mg/L or more.

In some embodiments, the isolated antigen-binding proteins of the present application can be measured for binding activity to a cell surface Siglec-15 protein by the FACS method. For example, it can be a cell surface human Siglec-15 protein, a cell surface monkey Siglec-15 protein and/or a cell surface murine Siglec-15 protein. For example, the isolated antigen-binding protein of the present application can bind to Siglec-15 or a functional fragment thereof with an EC₅₀ value of about 3.25 nM or less. For example, the isolated antigen-binding protein of the present application can bind to Siglec-15 or a functional fragment thereof with an EC₅₀ value of about 3.25 nM or less, about 3.0 nM or less, about 2.0 nM or less, about 1.9 nM or less, about 1.8 nM or less, about 1.7 nM or less, about 1.6 nM or less, about 1.5 nM or less, about 1.4 nM or less, about 1.3 nM or less, about 1.2 nM or less, about 1.1 nM or less, about 1.0 nM or less, about 0.9 nM or less, about 0.8 nM or less, about 0.7 nM or less, about 0.6 nM or less, about 0.5 nM or less, about 0.4 nM or less, about 0.3 nM or less, about 0.2 nM or less, or about 0.1 nM or less.

In some embodiments, the isolated antigen-binding protein of the present application can affect the ability of immune cells to proliferate. For example, the isolated antigen-binding protein of the present application can affect the function of the immune system; for example, the isolated antigen-binding protein of the present application can affect the proportion of immune cell subpopulations and/or the number of immune cells. For example, the immune cells may include lymphocytes, peripheral blood lymphocytes, peripheral blood mononuclear cells, PBMC, T cells, B cells, and/or NK cells. For example, the isolated antigen-binding protein of the present application can increase the proportion of immune cell subpopulations; for example, the isolated antigen-binding protein of the present application can increase the proportion of CD8⁺T cells; for example, the isolated antigen-binding protein of the present application can increase the proportion of CD4⁺T cells. For example, the isolated antigen-binding protein of the present application can increase the proportion of CD8⁺T cells in peripheral blood lymphocytes; for example, the isolated antigen-binding protein of the present application can increase the proportion of CD4⁺T cells in peripheral blood lymphocytes. For example, the isolated antigen-binding protein of the present application can increase the number of CD8⁺T cells by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, or more than about 90% compared to the group administered only blank control. For example, the isolated antigen-binding protein of the present application can increase the number of CD4⁺T cells by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, or more than about 90% compared to the group administered only blank control.

In some embodiments, the isolated antigen-binding proteins of the present can reverse the inhibitory effect of Siglec-15 protein on immune system function. For example, when the Siglec-15 protein is administered in vivo or in vitro and/or when the Siglec-15 protein is overexpressed in vivo or in vitro, the Siglec-15 protein can inhibit immune system function, e.g., the Siglec-15 protein can affect the proportion of a subpopulation of immune cells, inhibit the proliferation of immune cells, and/or reduce the number of immune cells. For example, an isolated antigen-binding protein of the present application can improve, reverse, restore, and/or activate the function of the immune system when the function of the immune system is inhibited by the Siglec-15 protein. For example, after the Siglec-15 protein inhibits the functions of the immune system and/or the number of immune cells, the isolated antigen-binding protein of the present application can increase the number of CD8⁺T cells by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, or more than about 90% compared to the group administered only blank control. For example, after the Siglec-15 protein inhibits the functions of the immune system and/or the number of immune cells, the isolated antigen-binding protein of the present application can increase the number of CD4⁺T cells by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, or more than about 90% compared to the group administered only blank control.

In some embodiments, the isolated antigen-binding protein of the disclosure can inhibit tumor growth. For example, the isolated antigen-binding protein is capable of inhibiting the growth of tumor, e.g., colon cancer, by about 23% or more tumor volume inhibition rate. For example, the isolated antigen-binding protein is capable of inhibiting tumor growth by about 20% or more, about 21% or more, about 22% or more, about 23% or more, about 24% or more, about 25% or more, about 26% or more, about 27% or more, about 28% or more, about 29% or more, about 30% or more, about 40% or more, or about 50% or more tumor volume inhibition rate compared to the group administered only blank control.

In some embodiments, the isolated antigen-binding proteins of the present application can affect the ability of cytokines secretion. For example, the secretion of a cytokine of the present application may include in vivo, ex vivo, and/or in vitro secretion. For example, the secretion of cytokines of the present application may include lipopolysaccharide-induced cytokine secretion. For example, lipopolysaccharide-induced cytokine secretion can refer to an increase in a amount of cytokines secreted in vivo and/or in vitro, an increase in the concentration of cytokines in culture fluid, and/or an increase in the concentration of cytokines in peripheral blood following the administration of lipopolysaccharide in vivo and/or in vitro. For example, the administration of the antigen-binding protein of the present application followed by lipopolysaccharide to a subject results in an increase in the amount of cytokine in the subject, compared to the administration of lipopolysaccharide alone or a blank followed by lipopolysaccharide. For example, compared to the administration of lipopolysaccharide alone or a blank followed by lipopolysaccharide, the antigen-binding proteins of the present application can increase the secretion of cytokines by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, more than about 90%, more than about 100%, about 150% or more, about 200% or more, about 250% or more, about 300% or more, about 350% or more, about 400% or more. For example, a cytokine of the present application may include an inflammatory factor, such as a factor associated with an inflammatory response or pro-inflammatory factors TNF-α and/or IL-6. For example, compared to the administration of lipopolysaccharide alone or a blank followed by lipopolysaccharide, the antigen-binding proteins of the present application can increase the secretion of TNF-α by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, more than about 90%, more than about 100%, about 150% or more, about 200% or more, about 250% or more, about 300% or more, about 350% or more, about 400% or more. For example, compared to the administration of lipopolysaccharide alone or a blank followed by lipopolysaccharide, the antigen-binding proteins of the present application can increase the secretion of IL-6 by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, more than about 90%, more than about 100%, about 150% or more, about 200% or more, about 250% or more, about 300% or more, about 350% or more, about 400% or more.

In one aspect, the present application provides a polypeptide molecule, a nucleic acid molecule, a vector, an immunoconjugate, a cell, and a pharmaceutical composition.

In another aspect, the present application provides a polypeptide molecule that may comprise the isolated antigen-binding protein of the application.

In some embodiments, the polypeptide molecule may comprise a fusion protein. In some embodiments, the polypeptide molecule can be a fusion protein. In some embodiments, the polypeptide molecules of the present application may comprise structures other than amino acids, for example, the polypeptide molecules of the present application may comprise nucleic acids, polysaccharides, lipids, small molecules, and any combination of the foregoing.

In another aspect, the present application provides an isolated nucleic acid molecule that can encode the isolated antigen-binding protein of the present application and/or the polypeptide of the present application. For example, it can be produced or synthesized by the following methods: (i) in vitro amplification, e.g., polymerase chain reaction (PCR) amplification, (ii) clonal recombination, (iii) purification, e.g., digestion and gel electrophoresis fractionation, or (iv) synthesis, e.g., chemical synthesis.

In another aspect, the present application provides a vector that may comprise the nucleic acid molecule of the present application. In addition, the vector can also comprise other genes, such as marker genes that allow for selection of the vector in an appropriate host cell and under appropriate conditions. In addition, the vector can also comprise expression control elements that allow for the proper expression of the coding region in an appropriate host. Such control elements are well known to those skilled in the art and can include, for example, promoters, ribosome binding sites, enhancers, and other control elements that regulate gene transcription or mRNA translation, etc. Expression of the genetic material element carried by the vector in a host cell can be achieved by transformation, transduction or transfection of the vector into the host cell. Such vectors can include, for example, plasmids, cosmids, viruses, phages or other vectors commonly used in, for example, genetic engineering. For example, the vector can be an expression vector. In addition, the vector can also include components that facilitate the vetor to enter into cells, including, but not limited to, viral particles, liposomes, or protein shells.

In another aspect, the present application also provides immunoconjugates that may comprise the isolated antigen-binding protein of the present application and/or the polypeptide of the present application. In some embodiments, the isolated antigen-binding protein or fragment thereof of the present application can be linked to another reagent, such as a chemotherapeutic agent, toxin, immunotherapeutic agent, imaging probe, spectroscopic probe, etc. The linkage can be formed via one or more covalent bonds, or non-covalent interactions, and may include chelation. A variety of linkers, which can be known in the art, can be used to form immunoconjugates. In addition, the immunoconjugate can be provided in a form of a fusion protein, which can be expressed from a polynucleotide encoding the immunoconjugate. The immunoconjugate can also comprise, for example, an antibody-drug ADC. In ADC, the antibody and therapeutic agent can be cross-linked by a linker, which can be a cleavable linker, for example an in vivo and/or in vitro enzyme-cleavable linker, for example a peptide linker, a disulfide linker, or a hydrazone linker.

In another aspect, the present application provides a cell that may comprise the isolated antigen-binding protein of the present application, the polypeptide molecule of the present application, the immunoconjugate of the present application, the nucleic acid molecule of the present application, or the vector of the present application. In some embodiments, each or every host cell may comprise one or one type of the nucleic acid molecules or vectors of the present application. In some embodiments, each or each host cell may comprise multiple (e.g., 2 or more) or multiple (e.g., 2 or more) nucleic acid molecules or vectors of the present application. For example, the vector of the present application can be introduced into the host cell, e.g., a eukaryotic cell, such as a cell from a plant, a fungal or yeast cell, etc. In some embodiments, the cell can be a bacterial cell (e.g., E. coli), a yeast cell, or other eukaryotic cell, such as a COS cell, a Chinese Hamster Ovary (CHO) cell, a CHO-K1 cell, a LNCAP cell, a HeLa cell, a 293T cell, a COS-1 cell, a SP2/0 cell, a NS0 cell, or a myeloma cell. The vectors of the present application can be introduced into the host be by methods known in the art, such as electroporation, lipofectine transfection, lipofectamin transfection, etc.

In another aspect, the present application also provides a pharmaceutical composition that may comprise the isolated antigen-binding protein of the present application, the polypeptide molecule of the present application, the immunoconjugate of the present application, the nucleic acid molecule of the present application, the vector of the present application and/or the cell of the present application, and optionally a pharmaceutically acceptable vehicle. In some embodiments, the pharmaceutical composition can further comprise a suitable formulation of one or more (pharmaceutically effective) adjuvants, stabilizers, excipients, diluents, solubilizers, surfactants, emulsifiers, and/or preservatives. Acceptable components of the compositions can be nontoxic to recipients at the dosages and concentrations employed. Pharmaceutical compositions of the present invention can include, but are not limited to, liquid, frozen, and lyophilized compositions.

In some embodiments, the pharmaceutical compositions can also contain more than one active compound, typically those with complementary activities that do not adversely affect each other. The type and effective amount of such drugs can depend, for example, on the amount and type of antagonist present in the formulation, as well as the clinical parameters of the subject.

In some embodiments, the pharmaceutically acceptable vehicle may include any and all solvents, dispersion media, coatings, isotonic and absorption delaying agents, and is generally safe and non-toxic, compatible with pharmaceutical administration.

In some embodiments, the pharmaceutical composition can be administered parenterally, transdermally, intraluminally, intraarterially, intrathecally and/or intranasally or injected directly into a tissue. For example, the pharmaceutical composition can be administered to a patient or subject by infusion or injection. In some embodiments, the administration of the pharmaceutical composition can be performed by various means, such as intravenous, intraperitoneal, subcutaneous, intramuscular, topical, or intradermal administration. In some embodiments, the pharmaceutical composition can be administered without interruption. Such uninterrupted (or continuous) administration can be accomplished by a small pump system worn by the patient to measure the flow of therapeutic agent into the patient.

In some embodiments, the present application provides a pharmaceutical combination. In some embodiments, the pharmaceutical combination of the present application may comprise the isolated Siglec-15 binding protein of the present application, the polypeptide comprising a Siglec-15 binding protein, the immunoconjugate of the present application, the nucleic acid molecule of the present application, the vector of the present application, the cell of the present application, and/or the pharmaceutical composition of the present application, and may comprise a suitable active substance having a prophylactic and/or therapeutic effect.

In some embodiments, wherein the pharmaceutical combination may comprise a first formulation including the isolated Siglec-15 binding protein of the present application and/or the polypeptide comprising the Siglec-15 binding protein and the pharmaceutically acceptable first carrier, and a second formulation including a suitable therapeutically active substance and a pharmaceutically acceptable second carrier.

In one aspect, the present application provides a kit. For example, wherein the kit may comprise the isolated Siglec binding protein of the present application, the polypeptide comprising the Siglec binding protein, the nucleic acid molecule encoding the isolated Siglec binding protein of the present application, the vector comprising the nucleic acid molecule encoding the isolated Siglec binding protein of the present application, the immunoconjugate comprising the isolated Siglec binding protein of the present application, the cell comprising the above substances, the composition of the present application, and/or the pharmaceutical combination of the present application. For example, the Siglec of the present application can be Siglec-15. In some embodiments, wherein the kit is used for detecting the presence and/or amount of Siglec-15 in a sample.

In one aspect, the present application provides a preparation method.

In another aspect, the present application provides a method of preparing the isolated Siglec-15 binding protein of the present application and/or the polypeptide comprising the Siglec-15 binding protein. The methods may comprise culturing the host cells of the present application under conditions capable of expressing the isolated Siglec-15 binding protein and/or the polypeptide comprising the Siglec-15 binding protein of the present application. For example, these methods can be performed by using an appropriate medium, an appropriate temperature, an appropriate culture time, etc., which are known to those of ordinary skill in the art.

Any method suitable for producing monoclonal antibody can be used to produce the antigen-binding proteins of the present application. For example, animals can be immunized with linked or naturally occurring Siglec-15 or fragments thereof. Suitable immunization methods can be used, including adjuvants, immunostimulants, repeated booster immunization, and one or more routes can be used. For example, hybridoma preparation methods can be used, in which splenocytes from immunized mice are obtained and fused with SP2/0 myeloma cells, and hybridoma cell strains are screened by HAT.

Any suitable form of Siglec-15 can be used as an immunogen (antigen) to generate non-human antibodies specific for Siglec-15, and the antibodies were screened for biological activity. For example, the priming immunogen can be full-length mature human Siglec-15, and may comprise native homodimers, or peptides containing single/multiple epitopes. The immunogen can be used alone or in combination with one or more immunogenicity-enhancing agent known in the art.

Chimeric human antibodies can be selected from any class of immunoglobulins, which may include IgM, IgD, IgG, IgA, and IgE. In the present application, the antibody can be an IgG antibody, and IgG1, IgG2, IgG3 or IgG4 subtypes can be used. Optimization of the necessary constant domain sequences to produce the desired biological activity can be achieved by screening antibodies using biological assays in the art. Likewise, any type of light chain can be used in the compounds and methods of the present application. For example, κ chains or variants thereof can be used in the compounds and methods of the present application.

In one aspect, the present application provides a method and use.

For example, the present application provides a method of detecting Siglec in a sample, which may comprise administering the isolated Siglec binding protein of the present application, administering the polypeptide comprising the Siglec binding protein, administering the nucleic acid molecule encoding the isolated Siglec binding protein of the present application, administering the vector comprising the nucleic acid molecule encoding the isolated Siglec binding protein of the present application, administering the immunoconjugate comprising the isolated Siglec binding protein of the present application, administering the cell comprising the above substances, administering the pharmaceutical composition of the present application, administering the pharmaceutical combination of the present application, and/or administering the kit of the present application. In some cases, the method of detecting Siglec in a sample can be an in vitro method. In some cases, the method of detecting Siglec in a sample can be a non-therapeutic method. In some cases, the method of detecting Siglec in a sample can be a non-diagnostic method. For example, the Siglec of the present application can be Siglec-15.

For example, the present application provides a method of affecting the binding of a Siglec or a functionally active fragment thereof to its ligand, which may comprise administering the isolated Siglec binding protein of the present application, administering the polypeptide comprising the Siglec binding protein, administering the nucleic acid molecule encoding the isolated Siglec binding protein of the present application, administering the vector comprising the nucleic acid molecule encoding the isolated Siglec binding protein of the present application, administering the immunoconjugate comprising the isolated Siglec binding protein of the present application, administering the cell comprising the above substances, administering the pharmaceutical composition of the present application, administering the pharmaceutical combination of the present application, and/or administering the kit of the present application. In some cases, the method of affecting binding of Siglec or a functionally active fragment thereof to its ligand can be an in vitro method. In some cases, the method of affecting binding of Siglec or a functionally active fragment thereof to its ligand can be a non-therapeutic method. In some cases, the method of affecting binding of Siglec or a functionally active fragment thereof to its ligand can be a non-diagnostic method. For example, the Siglec of the present application can be Siglec-15. For example, the ligands of the Siglec of the present application may comprise Leucine-rich repeat-containing protein 4C and sialylated Tn (sialyl-Tn). For example, affecting the binding of Siglec or a functionally active fragment thereof to its ligand can be manifested as a decrease in immune system function inhibition by Siglec by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, more than about 90%, about 100% or more, about 150% or more, about 200% or more, about 250% or more, about 300% or more, about 350% or more, or about 400% or more, compared to a blank control.

For example, the present application provides a method of affecting immune cell proliferation, which may comprise administering the isolated Siglec binding protein of the present application, administering the polypeptide comprising the Siglec binding protein, administering the nucleic acid molecule encoding the isolated Siglec binding protein of the present application, administering the vector comprising the nucleic acid molecule encoding the isolated Siglec binding protein of the present application, administering the immunoconjugate comprising the isolated Siglec binding protein of the present application, administering the cell comprising the above substances, administering the pharmaceutical composition of the present application, administering the pharmaceutical combination of the present application, and/or administering the kit of the present application. For example, the Siglec of the present application can be Siglec-15. In some cases, the method of affecting immune cell proliferation can be an in vitro method. In some cases, the method of affecting immune cell proliferation can be a non-therapeutic method. In some cases, the method of affecting immune cell proliferation can be a non-diagnostic method. For example, affecting immune cell proliferation according to the present application can be manifested as reducing the immune cell proliferation inhibition by Siglec-15. For example, the proportion of immune cell subpopulations and/or the number of immune cells can be affected. For example, the immune cells may include lymphocytes, peripheral blood lymphocytes, peripheral blood mononuclear cells and/or peripheral blood mononuclear cells. For example, the proportion of immune cell subpopulations can be increased; for example, the proportion of CD8⁺T cells can be increased; for example, the proportion of CD4⁺T cells can be increased. For example, the proportion of CD8⁺T cells in peripheral blood lymphocytes can be increased; for example, the proportion of CD4⁺T cells in peripheral blood lymphocytes can be increased. For example, the number of CD8⁺T cells can be increased by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, or more than about 90% compared to the blank control. For example, the number of CD4⁺T cells can be increased by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, or more than about 90% compared to the blank control.

For example, the present application provides a method of affecting cytokine secretion, which may comprise administering the isolated Siglec binding protein of the present application, administering the polypeptide comprising the Siglec binding protein, administering the nucleic acid molecule encoding the isolated Siglec binding protein of the present application, administering the vector comprising the nucleic acid molecule encoding the isolated Siglec binding protein of the present application, administering the immunoconjugate comprising the isolated Siglec binding protein of the present application, administering the cell comprising the above substances, administering the pharmaceutical composition of the present application, administering the pharmaceutical combination of the present application, and/or administering the kit of the present application. For example, the Siglec of the present application can be Siglec-15. In some cases, the method of affecting cytokine secretion can be an in vitro method. In some cases, the method of affecting cytokine secretion can be a non-therapeutic method. In some cases, the method of affecting cytokine secretion can be a non-diagnostic method. For example, affecting cytokine secretion can be manifested as affecting the of secretion lipopolysaccharide-induced cytokines, e.g., TNF-α and/or IL-6. For example, the secretion of TNF-α can be increased by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, more than about 90%, more than about 100%, about 150% or more, about 200% or more, about 250% or more, about 300% or more, about 350% or more, about 400% or more compared to the blank control. For example, the secretion of IL-6 can be increased by more than about 1%, more than about 2%, more than about 3%, more than about 4%, more than about 5%, more than about 7%, more than about 10%, more than about 15%, more than about 20%, more than about 25%, more than about 30%, more than about 35%, more than about 40%, more than about 45%, more than about 50%, more than about 60%, more than about 70%, more than about 80%, more than about 90%, more than about 100%, about 150% or more, about 200% or more, about 250% or more, about 300% or more, about 350% or more, about 400% or more compared to the blank control.

In another aspect, the present invention provides a use of the isolated Siglec binding protein of the present application, the polypeptide comprising the Siglec binding protein, the nucleic acid molecule encoding the isolated Siglec binding protein of the present application, the vector comprising the nucleic acid molecule encoding the isolated Siglec binding protein of the present application, the immunoconjugate comprising the isolated Siglec binding protein of the present application, the cell comprising the above substances, the composition of the present applicationand/or the pharmaceutical combination of the present application in the manufacture of a kit. For example, the kit can be used for detecting the presence and/or amount of Siglec in a sample. For example, the Siglec of the present application can be Siglec-15.

In one aspect, the present application provides a prophylactic and/or therapeutic use. Siglec-15 can serve as a new target, and an antibody targeting Siglec-15 can reverse the immune system function inhibition by Siglec-15. The available data can indicate that an antibody targeting Siglec-15 can activate an immune response, such that an antibody against Siglec-15 can be developed as a drug for preventing and/or treating a disease and/or condition, or an antibody against Siglec-15 can be used in a method for preventing and/or treating a disease and/or condition.

In another aspect, the present application provides a use of the isolated Siglec binding protein of the present application, the polypeptide comprising the Siglec binding protein, the nucleic acid molecule encoding the isolated Siglec binding protein of the present application, the vector comprising the nucleic acid molecule encoding the isolated Siglec binding protein of the present application, the immunoconjugate comprising the isolated Siglec binding protein of the present application, the cell comprising the above substances, the composition of the present application, the pharmaceutical combination of the present application and/or the kit of the present application in the manufacture of a medicament for the prevention and/or treatment of a disease and/or condition. For example, the disease and/or condition of the present application can be an immune-related disorder and/or a proliferative-related disorder. For example, the disease and/or condition of the present application can be a disease associated with abnormal expression of Siglec or dysfunction. For example, the disease and/or condition of the present application can be a Siglec-positive tumor. For example, the disease and/or condition of the present application can be a tumor with high expression of Siglec. In the present application, the disease and/or condition can be a Siglec-related disease and/or condition. For example, the Siglec of the present application may be Siglec-15.

In the present application, the disease and/or condition may include a tumor. In the present application, the disease and/or condition may include conditions with cellular overgrowth or proliferation. In the present application, the disease and/or condition may include proliferative diseases, neoplastic diseases, and/or immunological diseases. For example, the tumor of the present application can be a tumor and/or infection associated with associated with abnormal expression of Siglec protein or dysfunction. For example, the tumor of the present application can be a Siglec-positive tumor and/or can be a tumor with high expression of Siglec. In the present application, the tumor may include a solid tumor. In the present application, the disease and/or condition may include tumors in the digestive tract. In the present application, the disease and/or condition may include intestinal tumors. In the present application, the disease and/or condition may include colon cancer. For example, the Siglec of the present application may be Siglec-15.

In another aspect, the present application provides the isolated Siglec binding protein of the present application, the polypeptide comprising the Siglec binding protein, the nucleic acid molecule encoding the isolated Siglec binding protein of the present application, the vector comprising the nucleic acid molecule encoding the isolated Siglec binding protein of the present application, the immunoconjugate comprising the isolated Siglec binding protein of the present application, the cell comprising the above substances, the composition of the present application, the pharmaceutical combination of the present application and/or the kit of the present application, for the prevention and/or treatment of a disease and/or condition. For example, the disease and/or condition of the present application can be an immune-related disorder and/or a proliferative-related disorder. For example, the disease and/or condition of the present application can be a disease associated with abnormal expression of Siglec or dysfunction. For example, the disease and/or condition of the present application can be a Siglec-positive tumor. For example, the disease and/or condition of the present application can be a tumor with high expression of Siglec. In the present application, the disease and/or condition can be a Siglec-related disease and/or condition. For example, the Siglec of the present application can be Siglec-15.

In the present application, the disease and/or condition may include a tumor. In the present application, the disease and/or condition may include conditions with cellular overgrowth or proliferation. In the present application, the disease and/or condition may include proliferative diseases, neoplastic diseases, and/or immunological diseases. For example, the tumor of the present application can be a tumor and/or infection associated with associated with abnormal expression of Siglec protein or dysfunction. For example, the tumor of the present application can be a Siglec-positive tumor and/or can be a tumor with high expression of Siglec. In the present application, the tumor may include a solid tumor. In the present application, the disease and/or condition may include tumors in the digestive tract. In the present application, the disease and/or condition may include intestinal tumors. In the present application, the disease and/or condition may include colon cancer. For example, the Siglec of the present application can be Siglec-15.

In another aspect, the present application provides a method of preventing and/or treating a disease and/or condition, which may comprise administering to a subject in need thereof the isolated Siglec binding protein of the present application, the polypeptide comprising the Siglec binding protein, the nucleic acid molecule encoding the isolated Siglec binding protein of the present application, the vector comprising the nucleic acid molecule encoding the isolated Siglec binding protein of the present application, the immunoconjugate comprising the isolated Siglec binding protein of the present application, the cell comprising the above substances, the composition of the present application, the pharmaceutical combination of the present application, and/or the kit of the present application. For example, the disease and/or condition of the present application can be an immune-related disorder and/or a proliferative-related disorder. For example, the disease and/or condition of the present application can be a disease associated with abnormal expression of Siglec or dysfunction. For example, the disease and/or condition of the present application can be a Siglec-positive tumor. For example, the disease and/or condition of the present application can be a tumor with high expression of Siglec. In the present application, the disease and/or condition can be a Siglec-related disease and/or condition. For example, the Siglec of the present application may be Siglec-15.

In the present application, the disease and/or condition may include a tumor. In the present application, the disease and/or condition may include conditions with cellular overgrowth or proliferation. In the present application, the disease and/or condition may include proliferative diseases, neoplastic diseases, and/or immunological diseases. For example, the tumor of the present application can be a tumor and/or infection associated with associated with abnormal expression of Siglec protein or dysfunction. For example, the tumor of the present application can be a Siglec-positive tumor and/or can be a tumor with high expression of Siglec. In the present application, the tumor may include a solid tumor. In the present application, the disease and/or condition may include tumors in the digestive tract. In the present application, the disease and/or condition may include intestinal tumors. In the present application, the disease and/or condition may include colon cancer. For example, the Siglec of the present application can be Siglec-15.

In the present application, the administration can be carried out in different ways, such as intravenous, intratumoral, intraperitoneal, subcutaneous, intramuscular, topical, or intradermal administration.

Without intending to be bound by any theory, the following examples are intended merely to illustrate the binding proteins, methods of preparation, uses, etc., of the present application and are not intended to limit the scope of the present application.

### EXAMPLES

### Example 1 Preparation of recombinant cell line

An expression plasmid was constructed with the extracellular domain of human Siglec-15 (with amino acid sequence described in Uniprot, Q6ZMC9, 20-263) and the transmembrane domain of platelet-derived growth factor receptor (PDGFR) (with amino acid sequence described in P09619, 513-561) after codon-optimization; the lentivirus was packaged with expression plasmid to infect 293T cells; the expression of Siglec-15 was detected by antibiotics pressurization screening; the positive clones were selected and expanded to obtain recombinant cell line human S15ECD-293T overexpressing the amino acid sequence of extracellular domain of human Siglec-15. The monkey S15ECD-293T can be generated by referring to the above similar procedures.

Full length murine Siglec-15 (with amino acid sequence described in Uniprot, A7E1W8) was ligated with full length DAP12 amino acid sequence (O54885) (with amino acid sequence described in GSGEGRGSLLTCGDVEENPGP of SEQ ID NO: 41) by T2A, and subjected to codon optimization to construct an expression plasmid according to a conventional method; the lentivirus was packaged with the expression plasmid to infect CT26 cells to generate recombinant cell line murine S15-CT26 overexpressing full length murine Siglec-15.

### Example 2 Screening and maturation for Siglec-15 binding protein

### Phage screening for Siglec-15 binding protein

Human Siglec-15 antigen was subjected to two liquid phase screening rounds using antibody libraries. The library phages of 1×10¹³cfu were removed and pre-bound with 50 ml streptavidin magnetic beads for 30 min, and non-specifically bound phages were removed by specific adsorption using a magnetic rack. The procedure was repeated three times, a mixture of background-removed phages, 10 µg of biotinylated human Siglec-15 protein (abbreviation: HS-15 Biotin or human S-15 Biotin, Acrobiosystems, Cat. No.: SG5-H82E9), and 150 µg of streptavidin magnetic beads were incubated for 15 min at 37°C. The mixture was washed repeatedly with PBST for 14 times to remove unbound phages. The phages specifically binding to Siglec-15 were eluted with hydrochloric acid at a concentration of 100 mM, neutralized with Tris-HCl at pH 11, and infected exponentially-growing E. coli SS320, followed by the addition of helper phage M13KO7 at a final concentration of 1.5×10⁹ cfu 1 h after infection, and the mixture was incubated overnight at 32°C. Next day, the mixture was centrifuged at 15000 g for 10 min to collect supernatant, and 20% PEG/NaCl solution with a volume of 1/5 supernatant was added for precipitation at 4°C for 1 h; the precipitate was obtained by centrifugation at 15000 g for 20 min, and re-suspended with 1 ml of 1 × PBS for the second round of panning, in which the dosage was the same as the first round except that the dosage of antigen was reduced by half in each round. The enrichment of the two rounds of panning was determined by phage enzyme-linked immunosorbent assay (ELISA). Human Siglec-15 protein at a concentration of 2 ng/µL, was added at 100 µL/well for coating overnight at 4°C. The phages enriched from the second round of panning was added at 1 × 10¹¹ cfu/well, and the mixture incubated for 40 min at 37°C. Unbound phages were eluted with PBST, 1: 5000 dilution of HRP conjugated Mouse Anti-FLAG Monoclonal Antibody (Sigma, 8592) was added at 100 µL/well, the mixture was incubated at 37°C for 30 min, unbound phages were eluted with PBST, alkaline phosphatase chromogenic solution was added, and absorbance was read at 450 nm on a ELISA plate reader. The results are shown in Table 1:

**Table 1: ELISA data for binding of phage to Siglec-15**

| Coating antigen | OD₄₅₀ Absorption value |
|---|---|
| Human Siglec-15 | 0.4845 |
| Irrelevant protein | 0.0666 |

### Yeast display screening for Siglec-15 binding proteins

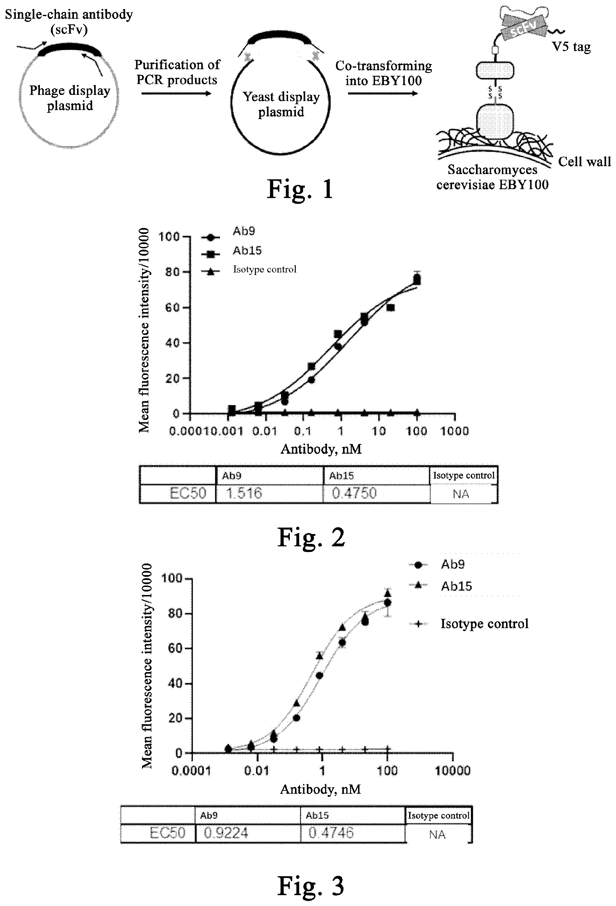

As shown in Fig. 1, according to the construction method of the yeast display of the present application, with a plasmid after panning a phage antibody library as a template, primers were designed to perform polymerase chain reaction (PCR) to amplify a single-chain antibody (scFv) gene, the scFv gene fragments amplified by the PCR were recovered and co-transformed with a yeast display plasmid into a Saccharomyces cerevisiae strain EBY100 (available from ATCC), and the scFv gene was inserted into the yeast display plasmid by homologous recombination in Saccharomyces cerevisiae, thereby displaying the single-chain antibody on the surface of the yeast cell wall. The yeast display single chain antibody library was named as JYYDL020. Library JYYDL020 was electroporated and cultured in 100 mL of SD-Trp medium (Clontech, Cat. No: 630308) overnight at 30°C, 225 rpm; 1.0×10⁸ bacteria were removed and resuspended in 20 mL YPGP liquid medium (2% galactose, 2% peptone, 1% yeast extract, 0.54% Na₂HPO₄, 0.86% NaH₂PO₄·H₂O) and cultured for 24 h at 20°C, 225 rpm, and stored in 4°C refrigerator for future use.

After the library was induced, the OD₆₀₀ of the bacterial solution was measured, with 1 OD calculated as 1.0×10⁷ cells, 4.0×10⁸ cells were removed and subjected to the first round of flow cytometry sorting, and the positive cell population combined with HS-15 Biotin was enriched. 2.0×10⁷ cells were removed and subjected to a second round of negative selection for irrelevant antigens to remove non-specifically bound cell populations. After sorting, an appropriate amount of cells were plated on SD-Trp solid medium and cultured at 30°C for 3 days.

### Identification of Siglec-15 binding protein yeast monoclones

One 48-well plate was selected from the first round of screening products of JYYDL020 antibody library for sequencing, numbered as Y11; two 48-well plates were selected from the second round of screening products of JYYDL020 for sequencing, numbered as Y14-Y15. After sequence analysis, single chain antibodies with a single sequence were obtained and the corresponding yeast monoclones were analyzed by flow cytometry staining according to Table 2.

**Table 2: Identification scheme for flow cytometry staining of single yeast colony**

| Scheme | Primary antibody | Secondary antibody |
|---|---|---|
| 1 | Human S-15 Biotin (30 nM) | Streptavidin-PE |
| | Mouse anti V5 | Goat Anti Mouse-647 |
| 2 | Murine S-15 Biotin (30 nM) | Streptavidin-PE |
| | Mouse anti V5 | Goat Anti Mouse-647 |
| 3 | Irrelevant antigen (30 nM) | Streptavidin-PE |
| | Mouse anti V5 | Goat Anti Mouse-647 |
| 4 | Monkey S-15-Fc (10 nM) | M&H Fc-APC |
| | | Anti V5 FITC |

In Scheme 1, the intensity of HS-15 Biotin bound to the cell population can be reflected by the PE mean fluorescence signal intensity (MFI). Similarly, in Scheme 2, Scheme 3 and Scheme 4, non-specific binding levels of murine S-15 Biotin (Acrobiosytems, Cat. No.: SG5-M52H7, biotinylated) and Monkey S-15-Fc (Acrobiosytems, Cat. No.: SG5-C5253) can be assessed, with results shown in Table 3.

**Table 3: Flow cytometric staining results and numbering of single yeast colony**

| Protein No. | Clone No. | Human S-15 binding signal | Monkey S-15 binding signal | Murine S-15 binding signal | Irrelevant antigen-binding signal |
|---|---|---|---|---|---|
| Ab0 | Y14C4 | 666 | 588 | 286 | 12.5 |

According to the staining results, clones that did not bind to human, monkey, and mouse were excluded, and the finally obtained sequences of single clones were constructed into plasmids for expression of Siglec-15 binding protein.

### Expression of Siglec-15 binding protein

The obtained full-length light and heavy chain protein sequences of the binding protein were subjected to codon-optimization, respectively, the codon-optimized DNA fragment (GENEWIZ) was subjected to gene synthesis, and the synthesized gene fragment was cloned into expression vector pcDNA3.4 (Life Technologies) in a form of IgG1. After expression plasmid amplification and plasmid extraction, ExpiCHO cells (ThermoFisher Scientific, A29133) were co-transfected with two plasmids, and transient antibody expression was performed according to the supplier's ExpiCHO expression system approach, with a procedure as follows: ExpiCHO cells were cultured to a density of 6×10⁶/mL in a total culture volume of 25 ml culture medium at 36.5°C and 8% carbon dioxide concentration, and 10 µg of antibody light and heavy chain expression plasmids were each transfected to the cells using ExpiFecta transfection reagents; one day after the transfection, 150 µL of each of 4 mL ExpiCHO enhancer and ExpiCHO adjuvant was added to the cultured cells, and the culture was continued for 9 days. The supernatant was obtained by centrifugation at 3500 rpm and 4°C. Magnetic beads (AmMagTM Protein A, Genscript, L00695) was mixed with the Siglec-15 binding protein expression supernatant of the present application, the mixture was incubated at room temperature for 2 h and washed twice with PBS, the supernatant was discarded, an appropriate amount of elution buffer (Protein G or A Sefinose TM Elution buffer, Sangon, C600481) was added, the mixture was mixed well, and then placed on a test tube rack for static incubation for 5 min, the magnetic beads were resuspended for 2-3 times during the incubation period, the elution was repeated for 2 times, after the elution, an appropriate amount of neutralizing solution 1M Tris-HCl, pH7.5 (Sangon, B548124) was added immediately to neutralize for later use, resulting the purified Siglec-15 binding protein, with the information shown in Table 4.

**Table 4: Data on expression of Siglec-15 full human binding protein**

| Protein No. | Theoretical isoelectric point | Expression quantity (mg/L) | Concentration (mg/mL) |
|---|---|---|---|
| Ab0 | 7.9 | 53 | 0.89 |

The purified Siglec-15 binding protein was subjected to affinity assay, physicochemical property analysis and cell binding, and in vitro functional activity assay, followed by affinity maturation.

### Construction of affinity maturation mutant library

The resulting Siglec-15 binding protein was subjected to affinity maturation to increase its affinity for human Siglec-15. A mutation library of each CDR was constructed by randomly mutating amino acids at an antigen-binding determinant(CDR) site of the Siglec-15 binding protein of the present application, and high-throughput screening of sequences with strong antigen-specific binding force was performed using yeast display technology. The amino acid sequences of the light and heavy chain variable regions of the Siglec-15 binding proteins of the present application can be encoded according to the Chothia numbering scheme, and the CDR can be defined according to Chothia. For the light chain variable region CDR1, CDR1, CDR3 and heavy chain variable region CDR1, CDR2, and CDR3 of the antibody molecule, NNK (N represents any nucleotide, and K represents G or T) mutation primers were designed to perform polymerase chain reaction (PCR) to amplify each CDR mutation library gene fragment, and each CDR mutation library gene fragment and yeast display plasmid were respectively transformed into Saccharomyces cerevisiae strain EBY100, so that each CDR mutation library was displayed on the yeast surface in the form of Fab. Meanwhile, the parent sequence of the Siglec-15 binding protein of the present application was displayed on the surface of yeast in the form of Fab, which was used as a control.

### Screening for affinity maturation mutant library

After the library was cultured and induced, 1×10⁹ cells were removed respectively and subjected to the first round of enrichment using the magnetic bead sorting system. Cells from each library were resuspended in 1×PBSA containing 1 nM HS-15 Biotin and incubated for 30 min. After washing, cells were mixed with Anti biotin beads and incubated for 10 min. Positive cells were collected by magnetic column. After the positive cells were cultured and induced again, 3.0×10⁷ cells were respectively removed and subjected to a second round of flow cytometry sorting using 1 nM HS-15 Biotin to collect a cell population with a high display level and a strong binding force to an antigen; according to the second round sorting, after incubation with 0.1 nM HS-15 Biotin antigen, the mixture was washed with 10 mL 1 × PBSA at room temperature for 2 h, and then subjected to a third round sorting; after sorting, the cells were plated on SD-Trp, Leu solid medium and cultured at 30°C for 3 days. Single clones were picked and subjected to sequencing analysis to obtain single sequences with mutations in each CDR of the light and heavy chains.

The resulting mutant plasmids of each CDR of light and heavy chains were mixed with parent plasmids, and the mixture was co-transformed into yeast strains to construct a combinatorial library of light and heavy chain mutations, numbered as JYYDL062. After the library was cultured and induced, 2.0×10⁷ cells were removed and subjected to flow cytometry sorting with 0.1 nM HS-15 Biotin to collect the cell population with high display level and strong binding force to antigen; after sorting, the cells were plated for growth, and single clones were picked for sequencing analysis.

### Identification of single clones

After sequencing analysis, the single clone with single sequence was identified by flow cytometry staining, incubated with 1 nM HS-15 Biotin for 30 min, respectively, and washed with 1 mL 1 × PBSA for 2 h at room temperature; after flow cytometry analysis, the fluorescence intensity of different clones binding to antigen was compared. Based on the staining results, clones with significantly improved binding strength to HS-15 Biotin were selected for further evaluation, and the evaluation results and sequence are shown in Tables 5A, 5B, 5C, and 5D.

**Table 5A: Staining evaluation and expression number of affinity-matured clones of Siglec-15 binding protein of the present application**

| Protein No. | Clone No. | Displaying MFI | Binding MFI | Binding force (Displaying MFI/ Binding MFI) *10000 |
|---|---|---|---|---|
| Ab0 | None | 2381 | 52.2 | 219 |
| Ab2 | YC32A6 | 3016 | 133 | 441 |
| Ab3 | YC33B2 | 2855 | 188 | 658 |
| Ab5 | YC33D1 | 2815 | 135 | 480 |
| Ab6 | YC33G4 | 2291 | 94.2 | 411 |
| Ab7 | YC33D5 | 2483 | 113 | 455 |
| Ab9 | YC29B3 | 4534 | 131 | 289 |
| Ab13 | YC45H4 | 2346 | 109 | 465 |
| Ab15 | YC44F5 | 2565 | 105 | 409 |

**Table 5B: Number (SEQ ID NO) of Siglec-15 binding protein sequence of the present application**

| Protein No. | HCDR1 | HCDR2 | HCDR3 | LCDR1 | LCDR2 | LCDR3 | VH | VL |
|---|---|---|---|---|---|---|---|---|
| Ab0 | 7 | 2 | 1 | 13 | 10 | 9 | 23 | 29 |
| Ab2 | 8 | 2 | 1 | 13 | 10 | 9 | 24 | 29 |
| Ab3 | 7 | 3 | 1 | 13 | 10 | 9 | 25 | 29 |
| Ab5 | 7 | 4 | 1 | 13 | 10 | 9 | 26 | 29 |
| Ab6 | 7 | 5 | 1 | 13 | 10 | 9 | 27 | 29 |
| Ab7 | 7 | 6 | 1 | 13 | 10 | 9 | 28 | 29 |
| Ab9 | 7 | 2 | 1 | 14 | 10 | 9 | 23 | 30 |
| Ab13 | 7 | 3 | 1 | 13 | 11 | 9 | 25 | 31 |
| Ab15 | 7 | 3 | 1 | 13 | 12 | 9 | 25 | 32 |

**Table 5C: Number (SEQ ID NO) of Siglec-15 binding protein sequence of the present application**

| Protein No. | HFR1 | HFR2 | HFR3 | HFR4 | LFR1 | LFR2 | LFR3 | LFR4 |
|---|---|---|---|---|---|---|---|---|
| Ab0 | 15 | 16 | 17 | 18 | 19 | 20 | 21 | 22 |
| Ab2 | 15 | 16 | 17 | 18 | 19 | 20 | 21 | 22 |
| Ab3 | 15 | 16 | 17 | 18 | 19 | 20 | 21 | 22 |
| Ab5 | 15 | 16 | 17 | 18 | 19 | 20 | 21 | 22 |
| Ab6 | 15 | 16 | 17 | 18 | 19 | 20 | 21 | 22 |
| Ab7 | 15 | 16 | 17 | 18 | 19 | 20 | 21 | 22 |
| Ab9 | 15 | 16 | 17 | 18 | 19 | 20 | 21 | 22 |
| Ab13 | 15 | 16 | 17 | 18 | 19 | 20 | 21 | 22 |
| Ab15 | 15 | 16 | 17 | 18 | 19 | 20 | 21 | 22 |

**Table 5D: Number (SEQ ID NO) of Siglec-15 binding protein sequence of the present application**

| | |
|---|---|
| Heavy chain constant region sequence | 39 |
| Light chain constant region sequence | 40 |

### Expression and affinity assay of Siglec-15 binding protein affinity-matured antibody

The Siglec-15 binding protein of the present application was expressed and purified by the above-mentioned ExpiCHO expression system of the present application to obtain the purified Siglec-15 binding protein, with the information shown in Table 6.

**Table 6: Data on expression of Siglec-15 binding protein of the present application**

| Protein No. | Theoretical isoelectric point | Yield (mg) | Expression quantity (mg/L) | Volume (mL) | Concentration (mg/mL) |
|---|---|---|---|---|---|
| Ab0 | 7.9 | 3.45 | 138 | 1.7 | 2.03 |
| Ab2 | 7.9 | 5.76 | 230 | 2.0 | 2.88 |
| Ab3 | 7.8 | 2.58 | 103 | 1.5 | 1.72 |
| Ab5 | 7.9 | 2.39 | 95 | 1.5 | 1.59 |
| Ab6 | 7.9 | 2.33 | 93 | 1.5 | 1.55 |
| Ab7 | 7.9 | 2.06 | 82 | 1.5 | 1.37 |
| Ab9 | 7.8 | 3.56 | 142 | 1.7 | 2.09 |
| Ab13 | 7.8 | 4.16 | 166 | 2.0 | 2.08 |
| Ab15 | 7.8 | 3.86 | 154 | 1.5 | 2.57 |

### Example 3: Affinity assay of Siglec-15 binding protein

Affinity of Siglec-15 binding protein of the present application to human Siglec-15 (Aero, Cat. No.: SG5-H52H3) was determined using Octet RED96e (Fortebio), both of the antigen and the Siglec-15 binding protein of the present application were diluted with 1×PBST (1×PBS: Sangon, B548117-0500; 0.02% Tween 20: Sigma-aldrich, P1379). The antigen is used at a concentration of 30 nM and the Siglec-15 binding protein of the present application is used at a concentration of 33.3 nM.

### Loading sample for test (Octet Data Acquisition 11.1.0.11)

First, a sample was added to a 96-well plate (Greiner bio-one, 655209) in a system of 200 µL/well. Software parameters were then set, the plate temperature was set to 30°C, and the frequency at which the standard kinetic signal was collected was 5.0 Hz. Next, the AHC sensor (Fortébio, Cat. No.: 18-0015) was pre-wetted with 1×PBST for 10 min, then the sample was loaded on the machine for detection, with each cycle containing the following steps: 1) immersing the sample in buffer for 60 s; 2) detecting whether the antigen has non-specific binding to the sensor; 3) regenerating in a 10 mM glycine solution at pH 1.7; 4) immersing in buffer for 60 s; 5) immobilizing the Siglec-15 binding protein of the present application on the sensor for 20 s; 6) immersing the sensor in buffer for 180 s; 7) binding the antigen to the Siglec-15 binding protein of the present application for 180 s; 8) dissociating the antigen with Siglec-15 binding protein of the present application for 10 min; and 9) regenerating the sensor.

### Data Analysis

The equilibrium dissociation constant (K_{D}) of the Siglec-15 binding protein of the present application was calculated by determining the binding rate constant (kon) and dissociation rate constant (kdis) of the antigen-Siglec-15 binding protein of the present application in a 1: 1 binding manner using Data Analysis 12.0 software fromx Fortebio. The results are shown in Tables 7A, 7B, and 7C below.

**Table 7A: First assay of Siglec-15 binding protein affinity**

| Protein No. | Response value | *K_{D}* (M) | kon(1/Ms) | kdis(1/s) |
|---|---|---|---|---|
| Ab0 | 0.2435 | 1.09E-10 | 2.98E+05 | 3.24E-05 |

**Table 7B: Second assay of Siglec-15 binding protein affinity**

| Protein No. | Response value | *K_{D}* (M) | kon(1/Ms) | kdis(1/s) |
|---|---|---|---|---|
| Ab2 | 0.2615 | 1.554E-10 | 4.48E+05 | 6.96E-05 |
| Ab3 | 0.288 | 4.44E-11 | 4.40E+05 | 1.95E-05 |
| Ab5 | 0.2768 | 7.60E-12 | 3.38E+05 | 2.57E-06 |
| Ab6 | 0.2851 | 8.434E-12 | 3.40E+05 | 2.87E-06 |
| Ab7 | 0.2839 | 5.084E-12 | 2.82E+05 | 1.44E-06 |
| Ab9 | 0.2592 | 8.735E-11 | 4.45E+05 | 3.88E-05 |
| Ab13 | 0.3054 | 6.807E-11 | 5.56E+05 | 3.79E-05 |
| Ab15 | 0.3612 | 7.298E-11 | 5.38E+05 | 3.93E-05 |

**Table 7C: Third assay of Siglec-15 binding protein affinity**

| Protein No. | Response value | *K_{D}* (M) | kon(1/Ms) | kdis(1/s) |
|---|---|---|---|---|
| Ab15 | 0.3426 | 9.161E-11 | 4.75E+05 | 4.35E-05 |
| Ab9 | 0.315 | 6.914E-11 | 5.19E+05 | 3.59E-05 |

### Example 4 Assay for cell binding activity of Siglec-15 binding protein

The purified Siglec-15 binding protein of the present application was identified by flow cytometry for its function to bind to human S15ECD-293T cells of the present application. Human S15ECD-293T cells prepared by the methods of the present application can be expanded to 90% confluence in 15 cm cell culture dishes, media were pipetted completely, the cells were washed once with PBS buffer (Thermo Fisher, 10010049), then treated with enzyme-free cell dissociation buffer (Versene solution, Thermo Fisher, 15040066) and harvested. The cells were washed twice with PBS buffer and counted, the desired quantity of cells was removed for centrifugation, the cells were resuspended to 2×10⁶ cells/ml with FACS buffer (PBS with 2% FBS). After incubation at room temperature for 15 min, the cell suspension was added to a U-shaped 96-well plate (Corning, 3798) at 50 µL/well. The Siglec-15 binding proteins of the present application were serially diluted in FACS buffer, the dilution was added to the above 96-well plate at 50 µL/well and incubated at 4°C for 1 h. The incubated mixture was centrifuged to discard the supernatant, the cells were washed once with FACS buffer, and 100 µL of a fluorescently (Alexa 488) labeled secondary antibody (Thermo Fisher, A-11013) was added to each well and incubated at 4°C for 1 h. The cells were washed once with FACS buffer, 100 µL of fixative (4% (v/v) paraformaldehyde) was added per well to resuspend the cells, and the cells were washed twice with FACS buffer after 10 min. The cells were resuspended in 30 µL/well FACS buffer, and detected and analyzed by flow cytometry intellycite plus (Sartorius). The function of the purified anti-human Siglec-15 binding protein in binding to murine S15-CT26 and monkey S15ECD-293T cells was identified with a similar method.

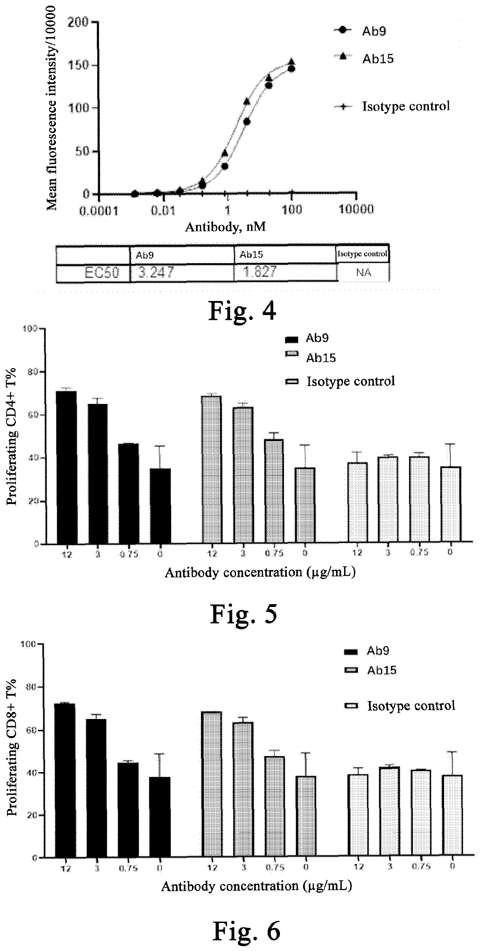

As shown in Figs. 2, 3 and 4, the Siglec-15 binding proteins of the present application can bind to Siglec-15 on the surface of a cell. Fig. 2 shows the results for the detection for binding of a Siglec-15 binding protein of the present application to human Siglec-15 on the surface of a cell. Fig. 3 shows the results for the detection for binding of a Siglec-15 binding protein of the present application to monkey Siglec-15 on the surface of a cell. Fig. 4 shows the results for the detection for binding of a Siglec-15 binding protein of the present application to murine Siglec-15 on the surface of a cell. EC₅₀ values (nM) of Ab9, Ab15 and isotype control of the present application for binding to human S15ECD-293T cells were 1.516, 0.4750 and NA (no data available), respectively; EC₅₀ values (nM) of Ab9, Ab15 and isotype control of the present application for binding to monkey S15ECD-293T cells in the present application were 0.9224, 0.4746 and NA (no data available), respectively; EC₅₀ values (nM) of Ab9, Ab15, and isotype control of the present application for binding to murine S15-CT26 cells in the present application were 3.247, 1.827, and NA (no data available), respectively.

### Example 5 Evaluation of physicochemical properties of Siglec-15 binding proteins

The physicochemical properties of the Siglec-15 binding protein of the present application can be determined with reference to the following methods.

### EC-HPLC Purity Analysis

(1) The sample was diluted to 1 mg/mL, mixed thoroughly, and centrifuged at 12000 rpm for 5 min, the supernatant was transferred to a sample bottle, and placed into a HPLC sample tray. The chromatographic conditions were set as follows: the column can be TSK G3000SWxl; the detection wavelength can be 280 nm; the column temperature can be 25°C; the sample chamber temperature can be 5°C; the flow rate can be 0.5 mL/min;
(2) The chromatographic column was equilibrated with mobile phase (200 mM phosphate buffer, pH 6.8), the sample was injected for analysis. The chromatographic software was used for data analysis. The peak area percentage of each peak was calculated by peak area normalization method.

### HIC-HPLC analysis

(1) The sample was diluted to 1 mg/ml, and centrifuged, and the supernatant was removed for assay. The chromatographic conditions were set as follows: the column can be MAbPac TM HIC-10; the detection wavelength can be 214 nm; the column temperature can be 30°C; the sample chamber temperature can be 5°C; the flow rate can be 0.8 mL/min;
(3) Gradient elution was performed with mobile phase A (50 mM phosphate buffer/1M ammonium sulfate, pH 7.0) and mobile phase B (50 mM phosphate buffer, pH 7.0), and the main peak retention time was recorded.

### Analysis of melting temperature (Tm) value

The test sample was diluted with sample buffer to 1 mg/mL, then according to the instructions of Protein Thermal Shift Starter Kit (ThermoFisher), to a PCR tube was added 13 µL of test sample solution, followed by 5 µL of Protein Thermal shift Buffer and 2 µL of 10× staining solution, resulting a reaction volume of 20 µL, the mixture was mixed thoroughly, and centrifuged at 12000 rpm for 5 min to remove air bubbles. The test sample was placed in the PCR instrument for sample analysis, and the Tm value of sample was recorded.

### iCIEF analysis

The sample solution was added into the following thoroughly mixed system: 1% methylcellulose (MC) 70 µL, 5 M urea 80 µL, amphoteric electrolyte Pharmalyte pH 3-10 8 µL, pI marker 5.5 2 µL, pI marker 9.5 2 µL. The system was supplemented with appropriate volume of ultrapure water to 200 µL, and the mixture was mixed thoroughly, then centrifuged to remvoe the supernatant for analysis. After the analysis, the result file was imported into ChromPerfect software for spectrum integration processing and calculation of the isoelectric point of each peak as well as the percentage of each peak. The Siglec-15 binding protein of the present application can have a purity of greater than or equal to 98% and a melting temperature Tm ranging from 86°C to 92°C, and can have good thermal stability for following development.

### Example 6 Inhibitory effect of Siglec-15 binding protein of the present application on proliferation of T cells in human PBMC

CD3 Monoclonal Antibody (OKT3) (Thermo Fisher, Cat. No.: 16-0037-85) was diluted to 0.03 µg/ml with PBS to coat the cell culture plate (Corning, code 3599) (100 µ L/well) overnight at 4°C. The next day, the Siglec-15 binding protein of the present application was diluted with culture medium (RPMI1640 + 10%FBS): the highest final concentration was 12 µg/mL (prepared concentration of 48 µg/mL), 4-fold serial dilution (3 gradient concentrations + 1 concentration of 0); human Siglec-15 Fc protein (Sino Biological Inc. Cat. No.: 13976-H02H) was diluted with culture medium to a final concentration of 5 µg/mL (prepared concentration of 20 µg/mL). The coating solution in the coating plate was pipetted and discarded, the plate was washed twice with PBS, to the plate was added 50 µL of the prepared Siglec-15 binding protein of the present application, followed by 50 µL of the prepared human Siglec-15 Fc protein. Fresh human peripheral blood lymphocytes (AllCells, Cat. No.: PB004-C) were then labeled, centrifuged at 400 × g for 10 min, the cell pellet was resuspended in PBS to adjust to a cell density of 1 × 10⁷ cells/mL, then an equal volume of 2 µM CFSE (Thermo Fisher, Cat. No.: C34554) was added, and mixed immediately after the addition, the mixture was subjected to 37°C water bath for 5 min, and terminated with 10 times volume of PBS + 10% FBS. The cells were then centrifuged at 400× g for 10 min, washed twice with PBS + 10% FBS, and resuspended to 2×10⁶ cells/mL with the medium. Then the cells labeled with CFSE were added into the culture plate at 100 µL/well, i.e., the number of cells per well was 2 × 10⁵ cells/well; the cell culture plates were incubated in a 37°C and 5% carbon dioxide cell incubator for 3 days, the cells were centrifuged at 300 × g for 5 min and washed once with pre-cooled PBS + 2% FBS, the cell pellet was resuspended with 100 µL of PBS + 2% FBS, 2 µL of Fc blocking reagent was added to each well, then the plate was placed in a 4°C refrigerator to block 30 min, followed by blending, the cells in each well were divided into 2 aliquots, wherein to one aliquot was added 5 µL of APC Mouse Anti-Human CD4 antibody (BD, Cat. No.: 555349), and to another aliquot was added 5 µL of APC Mouse Anti-Human CD8 antibody (BD, Cat. No.: 555369); after incubation for 1 h in a 4°C refrigerator, the cells were washed twice with pre-cooled PBS + 2% FBS, subsequently resuspended with 30 µL PBS + 2% FBS, and loaded on a iQue flow cytometer (intellycite plus, Sartorius) for detection. Flow results were analyzed with Flow jo V10 and data were processed with GraphPad Prism software.

Fig. 5 shows the results for the detection for reversing the proliferation inhibitory effect of Siglec-15 protein on CD4⁺T cells by Siglec-15 binding protein of the present application. Fig. 6 shows the results for the detection for reversing the proliferation inhibitory effect of Siglec-15 protein on CD8⁺T cells by Siglec-15 binding protein of the present application. As shown in Figs. 5 and 6, the Siglec-15 binding proteins of the present application can reverse the proliferative inhibitory effect of Siglec-15 protein on CD4⁺ and/or CD8⁺T cells.

### Example 7 Evaluation of the anti-tumor activity of the Siglec-15 binding proteins of the present application in a mouse tumor model

CT-26.WT (available from ATCC) mouse colon cancer cells were expanded in vitro while BMDM (macrophages derived from bone marrow) were cultured. BMDM was removed from bone marrow of femur and tibia of BALB/c mouse (available from Beijing Vital River Laboratory Animal Technology Co., Ltd., 7-8 weeks old, female), cultured with RPMI-1640 medium supplemented with 10% fetal bovine serum and 20 ng/ml macrophage colony stimulating factor (M-CSF), then collected after 7 days, and inoculated subcutaneously into BALB/c mouse in combination with CT-26.WT cells in an amount of 1×10⁵ for each mouse. Twelve days after inoculation, when the average tumor volume of mice reached 100 mm³, the mice were randomly divided into groups of 8 mice each according to tumor volume and body weight, and administered on the same day. G1 was administered with isotype control human IgG, G2 was administered with Ab9 of the present application, and G3 was administered with Ab15. Each group of mice was administered with 10 mg/kg intraperitoneally, twice a week.

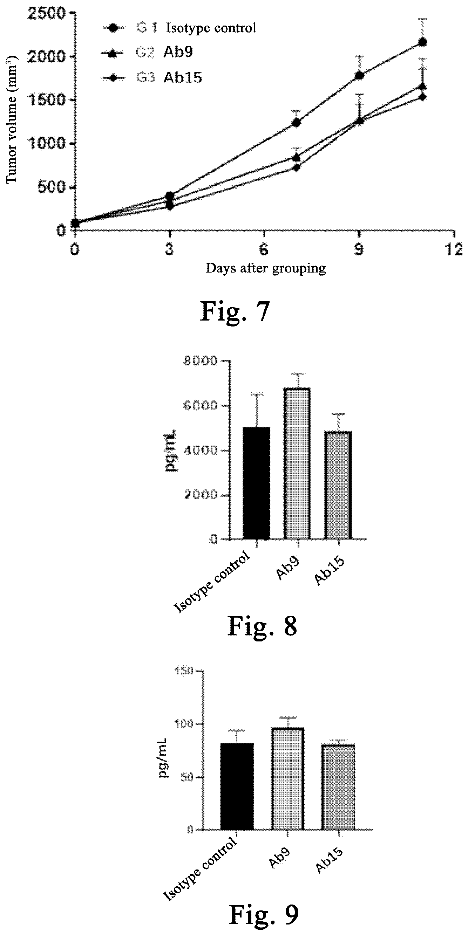

As shown in Fig. 7, on Day 11 after grouping, the mean tumor volume was 2170 ± 269 mm³ in group G1; the mean tumor volume was 1677 ± 191 mm³ and tumor inhibition rate was 23% in group G2; the mean tumor volume was 1540 ± 437 mm³ and the tumor inhibition rate was 29% in group G3. It was shown that the Siglec-15 binding protein of the present application had a inhibitory effect on tumor growth.

### Example 8 Enhancement of LPS-induced cytokine secretion by Siglec-15 binding protein of the present application

BALB/c mice (commercially available from Beijing Vital River Laboratory Animal Technology Co., Ltd., 7-8 weeks old, male) were randomly divided into groups of 6 mice each according to body weight. Group G1 was administered with isotype control human IgG, group G2 was administered with Ab9 of the present application, and group G3 was administered with Ab15. Each group of mice was administered with 10 mg/kg intraperitoneally, recorded as 0 h; 2 h after administration, mice were intraperitoneally injected with lipopolysaccharide LPS dissolved in normal saline, at a dose of 200 µg/kg. 2 h after LPS modeling, animals in all groups were anesthetized via inhalation of isoflurane at a concentration of 2-5%, and 200 µL of whole blood was collected through orbit; 6 h after LPS modeling, animals in all groups were anesthetized via inhalation of isoflurane at a concentration of 2-5%, and the maximum amount of peripheral blood was collected through orbit, followed by standing at room temperature for 30-60 min, and centrifuged at 2000g for 10 min at 4°C to separate serum to detect cytokines TNF-α and IL-6. All experimental animals were euthanized with an overdose of carbon dioxide.

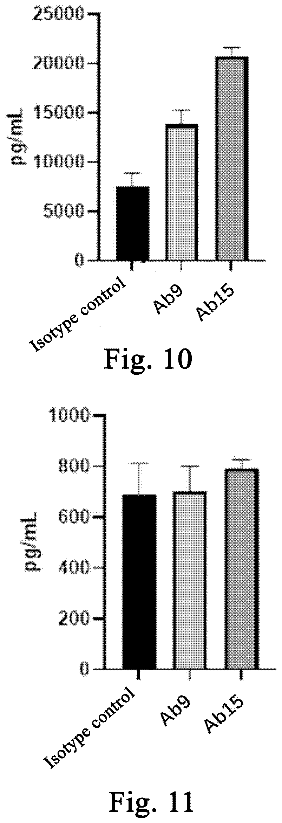

Fig. 8 shows the results for the detection for affecting secretion level of TNF-α (TNF-alpha) by Siglec-15 binding proteins of the present application 2 h after LPS injection. Fig. 9 shows the results for the detection for affecting secretion level of TNF-α (TNF-alpha) by Siglec-15 binding proteins of the present application 6 h after LPS injection. Fig. 10 shows the results for the detection for affecting secretion level of IL-6 by Siglec-15 binding proteins of the present application 2 h after LPS injection. Fig. 11 shows the results for the detection for affecting secretion level of IL-6 by Siglec-15 binding proteins of the present application 6 h after LPS injection. As shown in Figs. 8, 9, 10, and 11, 2 h after LPS injection, TNF-α (TNF-alpha) was significantly increased in group G2 relative to isotype control group, and basically returned to the same level after 6 h; 2 h after LPS injection, IL-6 was significantly increased in all administration groups relative to the isotype control group and returned to baseline levels after 6 h. It was shown that the Siglec-15 binding protein of the present application promoted the release of inflammatory factors in an LPS-induced acute inflammation model.

The foregoing detailed description is provided by way of explanation and examples, and is not intended to limit the scope of the appended claims. Various changes of the presently recited embodiments listed in the present invention are obvious to those of ordinary skills in the art, and reserved within the scope of the appended claims and the equivalent solutions thereof.

## Claims

1. An isolated antigen-binding protein, comprising HCDR3 comprising an amino acid sequence as set forth in SEQ ID NO: 1.

2. The isolated antigen-binding protein according to claim 1, comprising HCDR2 comprising an amino acid sequence as set forth in SEQ ID NO: 33.

3. The isolated antigen-binding protein according to any one of claims 1-2, comprising HCDR2 comprising an amino acid sequence as set forth in SEQ ID NO: 2, SEQ ID NO: 3, SEQ ID NO: 4, SEQ ID NO: 5, or SEQ ID NO: 6.

4. The isolated antigen-binding protein according to any one of claims 1-3, comprising HCDR1 comprising an amino acid sequence as set forth in SEQ ID NO: 34.

5. The isolated antigen-binding protein according to any one of claims 1-4, comprising HCDR1 comprising an amino acid sequence as set forth in SEQ ID NO: 7 or SEQ ID NO: 8.

6. The isolated antigen-binding protein according to any one of claims 1-5, comprising LCDR3 comprising an amino acid sequence as set forth in SEQ ID NO: 9.

7. The isolated antigen-binding protein according to any one of claims 1-6, comprising LCDR2 comprising an amino acid sequence as set forth in SEQ ID NO: 35.

8. The isolated antigen-binding protein according to any one of claims 1-7, comprising LCDR2 comprising an amino acid sequence as set forth in SEQ ID NO: 10, SEQ ID NO: 11, or SEQ ID NO: 12.

9. The isolated antigen-binding protein according to any one of claims 1-8, comprising LCDR1 comprising an amino acid sequence as set forth in SEQ ID NO: 36.

10. The isolated antigen-binding protein according to any one of claims 1-9, comprising LCDR1 comprising an amino acid sequence as set forth in SEQ ID NO: 13 or SEQ ID NO: 14.

11. The isolated antigen-binding protein according to any one of claims 1-10, comprising H-FR1, wherein the C-terminus of the H-FR1 is directly or indirectly linked to the N-terminus of the HCDR1, and the H-FR1 comprises an amino acid sequence as set forth in SEQ ID NO: 15.

12. The isolated antigen-binding protein according to any one of claims 1-11, comprising H-FR2, wherein the H-FR2 is located between the HCDR1 and the HCDR2, and the H-FR2 comprises an amino acid sequence set forth in SEQ ID NO: 16.

13. The isolated antigen-binding protein according to any one of claims 1-12, comprising H-FR3, wherein the H-FR3 is located between the HCDR2 and the HCDR3, and the H-FR3 comprises an amino acid sequence set forth in SEQ ID NO: 17.

14. The isolated antigen-binding protein according to any one of claims 1-13, comprising H-FR4, wherein the N-terminus of the H-FR4 is directly or indirectly linked to the C-terminus of the HCDR3, and the H-FR4 comprises an amino acid sequence as set forth in SEQ ID NO: 18.

15. The isolated antigen-binding protein according to any one of claims 1-14, comprising L-FR1, wherein the C-terminus of the L-FR1 is directly or indirectly linked to the N-terminus of the LCDR1, and the L-FR1 comprises an amino acid sequence as set forth in SEQ ID NO: 19.

16. The isolated antigen-binding protein according to any one of claims 1-15, comprising L-FR2, wherein the L-FR2 is located between the LCDR1 and the LCDR2, and the L-FR2 comprises an amino acid sequence as set forth in SEQ ID NO: 20.

17. The isolated antigen-binding protein according to any one of claims 1-16, comprising L-FR3, wherein the L-FR3 is located between the LCDR2 and the LCDR3, and the L-FR3 comprises an amino acid sequence as set forth in SEQ ID NO: 21.

18. The isolated antigen-binding protein according to any one of claims 1-17, comprising L-FR4, wherein the N-terminus of the L-FR4 is directly or indirectly linked to the C-terminus of the LCDR3, and the L-FR4 comprises an amino acid sequence as set forth in SEQ ID NO: 22.

19. The isolated antigen-binding protein according to any one of claims 1-18, comprising HCDR3 comprising an amino acid sequence as set forth in SEQ ID NO: 1, HCDR2 comprising an amino acid sequence as set forth in SEQ ID NO: 33, and HCDR1 comprising an amino acid sequence as set forth in SEQ ID NO: 34.

20. The isolated antigen-binding protein according to any one of claims 1-19, comprising HCDR3 comprising an amino acid sequence as set forth in SEQ ID NO: 1, HCDR2 comprising an amino acid sequence as set forth in SEQ ID NO: 2, SEQ ID NO: 3, SEQ ID NO: 4, SEQ ID NO: 5, or SEQ ID NO: 6, and HCDR1 comprising an amino acid sequence as set forth in SEQ ID NO: 7 or SEQ ID NO: 8.

21. The isolated antigen-binding protein according to any one of claims 1-20, comprising LCDR3 comprising an amino acid sequence as set forth in SEQ ID NO: 9, LCDR2 comprising an amino acid sequence as set forth in SEQ ID NO: 35, and LCDR1 comprising an amino acid sequence as set forth in SEQ ID NO: 36.

22. The isolated antigen-binding protein according to any one of claims 1-21, comprising LCDR3 comprising an amino acid sequence as set forth in SEQ ID NO: 9, LCDR2 comprising an amino acid sequence as set forth in SEQ ID NO: 10, SEQ ID NO: 11 or SEQ ID NO: 12, and LCDR1 comprising an amino acid sequence as set forth in SEQ ID NO: 13 or SEQ ID NO: 14.

23. The isolated antigen-binding protein according to any one of claims 1-22, comprising HCDR3 comprising an amino acid sequence as set forth in SEQ ID NO: 1, HCDR2 comprising an amino acid sequence as set forth in SEQ ID NO: 33, HCDR1 comprising an amino acid sequence as set forth in SEQ ID NO: 34, LCDR3 comprising an amino acid sequence as set forth in SEQ ID NO: 9, LCDR2 comprising an amino acid sequence as set forth in SEQ ID NO: 35, and LCDR1 comprising an amino acid sequence as set forth in SEQ ID NO: 36.

24. The isolated antigen-binding protein according to any one of claims 1-23, comprising HCDR3 comprising an amino acid sequence as set forth in SEQ ID NO: 1, HCDR2 comprising an amino acid sequence as set forth in SEQ ID NO: 2, SEQ ID NO: 3, SEQ ID NO: 4, SEQ ID NO: 5, or SEQ ID NO: 6, HCDR1 comprising an amino acid sequence as set forth in SEQ ID NO: 7 or SEQ ID NO: 8, LCDR3 comprising an amino acid sequence as set forth in SEQ ID NO: 9, LCDR2 comprising an amino acid sequence as set forth in SEQ ID NO: 10, SEQ ID NO: 11, or SEQ ID NO: 12, and LCDR1 comprising an amino acid sequence as set forth in SEQ ID NO: 13 or SEQ ID NO: 14.

25. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 2, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 10, and LCDR1 comprising an amino acid sequence as set forth in SEQ ID NO: 13.

26. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 2, HCDR1 comprising an amino acid sequence as set forth in SEQ ID NO: 8, LCDR3 comprising an amino acid sequence as set forth in SEQ ID NO: 9, LCDR2 comprising an amino acid sequence as set forth in SEQ ID NO: 10, and LCDR1 comprising an amino acid sequence as set forth in SEQ ID NO: 13.

27. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 3, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 10, and LCDR1 comprising an amino acid sequence as set forth in SEQ ID NO: 13.

28. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 4, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 10, and LCDR1 comprising an amino acid sequence as set forth in SEQ ID NO: 13.

29. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 5, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 10, and LCDR1 comprising an amino acid sequence as set forth in SEQ ID NO: 13.

30. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 6, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 10, and LCDR1 comprising an amino acid sequence as set forth in SEQ ID NO: 13.

31. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 2, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 10, and LCDR1 comprising an amino acid sequence as set forth in SEQ ID NO: 14.

32. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 3, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 11, and LCDR1 comprising an amino acid sequence as set forth in SEQ ID NO: 13.

33. The isolated antigen-binding protein according to any one of claims 1-24, comprising HCDR3 comprising an amino acid sequence as set forth in SEQ ID NO: 1, HCDR2 comprising an amino acid sequence as set forth in SEQ ID NO: 3, HCDR1 comprising an amino acid sequence as set forth in SEQ ID NO: 7, LCDR3 comprising an amino acid sequence as set forth in SEQ ID NO: 9, LCDR2 comprising an amino acid sequence as set forth in SEQ ID NO: 12, and LCDR1 comprising an amino acid sequence as set forth in SEQ ID NO: 13.

34. The isolated antigen-binding protein according to any one of claims 1-33, comprising a heavy chain variable region (VH) comprising an amino acid sequence as set forth in SEQ ID NO: 37.

35. The isolated antigen-binding protein according to any one of claims 1-34, comprising VH comprising an amino acid sequence as set forth in SEQ ID NO: 23, SEQ ID NO: 24, SEQ ID NO: 25, SEQ ID NO: 26, SEQ ID NO: 27, or SEQ ID NO: 28.

36. The isolated antigen-binding protein according to any one of claims 1-35, comprising a light chain variable region (VL) comprising an amino acid sequence as set forth in SEQ ID NO: 38.

37. The isolated antigen-binding protein according to any one of claims 1-36, comprising VL comprising an amino acid sequence as set forth in SEQ ID NO: 29, SEQ ID NO: 30, SEQ ID NO: 31, or SEQ ID NO: 32.

38. The isolated antigen-binding protein according to any one of claims 1-37, comprising VH comprising an amino acid sequence as set forth in SEQ ID NO: 37, and VL comprising an amino acid sequence as set forth in SEQ ID NO: 38.

39. The isolated antigen-binding protein according to any one of claims 1-38, comprising VH comprising an amino acid sequence as set forth in SEQ ID NO: 23, SEQ ID NO: 24, SEQ ID NO: 25, SEQ ID NO: 26, SEQ ID NO: 27, or SEQ ID NO: 28, and VL comprising an amino acid sequence as set forth in SEQ ID NO: 29, SEQ ID NO: 30, SEQ ID NO: 31, or SEQ ID NO: 32.

40. The isolated antigen-binding protein according to any one of claims 1-39, comprising VH comprising an amino acid sequence as set forth in SEQ ID NO: 23, and VL comprising an amino acid sequence as set forth in SEQ ID NO: 29.

41. The isolated antigen-binding protein according to any one of claims 1-39, comprising VH comprising an amino acid sequence as set forth in SEQ ID NO: 24, and VL comprising an amino acid sequence as set forth in SEQ ID NO: 29.

42. The isolated antigen-binding protein according to any one of claims 1-39, comprising VH comprising an amino acid sequence as set forth in SEQ ID NO: 25, and VL comprising an amino acid sequence as set forth in SEQ ID NO: 29.

43. The isolated antigen-binding protein according to any one of claims 1-39, comprising VH comprising an amino acid sequence as set forth in SEQ ID NO: 26, and VL comprising an amino acid sequence as set forth in SEQ ID NO: 29.

44. The isolated antigen-binding protein according to any one of claims 1-39, comprising VH comprising an amino acid sequence as set forth in SEQ ID NO: 27, and VL comprising an amino acid sequence as set forth in SEQ ID NO: 29.

45. The isolated antigen-binding protein according to any one of claims 1-39, comprising VH comprising an amino acid sequence as set forth in SEQ ID NO: 28, and VL comprising an amino acid sequence as set forth in SEQ ID NO: 29.

46. The isolated antigen-binding protein according to any one of claims 1-39, comprising VH comprising an amino acid sequence as set forth in SEQ ID NO: 23, and VL comprising an amino acid sequence as set forth in SEQ ID NO: 30.

47. The isolated antigen-binding protein according to any one of claims 1-39, comprising VH comprising an amino acid sequence as set forth in SEQ ID NO: 25, and VL comprising an amino acid sequence as set forth in SEQ ID NO: 31.

48. The isolated antigen-binding protein according to any one of claims 1-39, comprising VH comprising an amino acid sequence as set forth in SEQ ID NO: 25, and VL comprising an amino acid sequence as set forth in SEQ ID NO: 32.

49. The isolated antigen-binding protein according to any one of claims 1-48, comprising an antibody heavy chain constant region.

50. The isolated antigen-binding protein of claim 49, wherein the antibody heavy chain constant region comprises a heavy chain constant region derived from a human antibody.

51. The isolated antigen-binding protein according to any one of claims 49-50, wherein the antibody heavy chain constant region comprises a heavy chain constant region derived from an IgG.

52. The isolated antigen-binding protein according to any one of claims 49-51, wherein the antibody heavy chain constant region comprises a heavy chain constant region derived from an IgG1.

53. The isolated antigen-binding protein according to any one of claims 1-52, comprising an antibody light chain constant region.

54. The isolated antigen-binding protein of claim 53, wherein the antibody light chain constant region comprises a light chain constant region derived from a human antibody.

55. The isolated antigen-binding protein according to any one of claims 53-54, wherein the antibody light chain constant region comprises a constant region derived from an Igκ.

56. The isolated antigen-binding protein according to any one of claims 1-55, including an antibody or an antigen-binding fragment thereof.

57. The isolated antigen-binding protein of claim 56, wherein the antibody includes a monoclonal antibody.

58. The isolated antigen-binding protein according to any one of claims 56-57, wherein the antibody is selected from one or more of the groups consisting of a murine antibody, a chimeric antibody, a humanized antibody, and a fully human antibody.

59. The isolated antigen-binding protein according to any one of claims 56-58, wherein the antigen-binding fragment is selected from one or more of the groups consisting of Fab, Fab', a Fv fragment, F(ab')₂, F(ab)₂, scFv, di-scFv, VHH, and dAb.

60. The isolated antigen-binding protein according to any one of claims 1-59, which is capable of specifically binding to a sialic acid-binding immunoglobulin-like lectin (Siglec) or a functionally active fragment thereof.

61. The isolated antigen-binding protein of claim 60, wherein the Siglec includes Siglec-15.

62. The isolated antigen-binding protein according to any one of claims 60-61, wherein the Siglec includes human Siglec, monkey Siglec, and/or murine Siglec.

63. The isolated antigen-binding protein according to any one of claims 1-62, having the ability to bind to Siglec-15 or a functional fragment thereof with a K_{D} value of about 1.56E-10 M or less.

64. The isolated antigen-binding protein according to any one of claims 1-63, having the ability to bind to Siglec-15 or a functional fragment thereof with an EC₅₀ value of about 3.25 nM or less.

65. The isolated antigen-binding protein of claim 64, wherein the Siglec-15 or functional fragment thereof includes Siglec-15 or a functional fragment thereof expressed on a cell.

66. The isolated antigen-binding protein according to any one of claims 1-65, having the ability to affect immune cells proliferation.

67. The isolated antigen-binding protein of claim 66, wherein the affecting immune cells proliferation includes reducing the Siglec-15-associated immune cell proliferation inhibition.

68. The isolated antigen-binding protein according to any one of claims 66-67, wherein the immune cells include immune cells derived from human.

69. The isolated antigen-binding protein according to any one of claims 66-68, wherein the immune cells include lymphocytes.

70. The isolated antigen-binding protein according to any one of claims 66-69, wherein the immune cells include peripheral blood lymphocytes.

71. The isolated antigen-binding protein according to any one of claims 66-70, wherein the immune cells include T cells.

72. The isolated antigen-binding protein according to any one of claims 66-71, wherein the immune cells include CD4+ and/or CD8+ cells.

73. The isolated antigen-binding protein according to any one of claims 1-72, having tumor inhibition ability.

74. The isolated antigen-binding protein of claim 73, wherein the tumor inhibition ability includes affecting the tumor volume.

75. The isolated antigen-binding protein according to any one of claims 73-74, having the ability to inhibit a tumor with a tumor volume inhibition rate of about 23% or more.

76. The isolated antigen-binding protein according to any one of claims 1-75, having the ability to affect cytokine secretion.

77. The isolated antigen-binding protein of claim 76, wherein the secretion includes in vivo, ex vivo, and/or in vitro secretion.

78. The isolated antigen-binding protein according to any one of claims 76-77, wherein the cytokine secretion includes lipopolysaccharide-induced cytokine secretion.

79. The isolated antigen-binding protein according to any one of claims 76-78, wherein the cytokine includes an inflammatory factor.

80. The isolated antigen-binding protein according to any one of claims 76-79, wherein the cytokine includes TNF-α and/or IL-6.

81. A polypeptide, comprising the isolated antigen-binding protein according to any one of claims 1-80.

82. The polypeptide of claim 81, comprising a fusion protein.

83. A nucleic acid molecule encoding the isolated antigen-binding protein according to any one of claims 1-80 and/or the polypeptide according to any one of claims 81-82.

84. A vector comprising the nucleic acid molecule of claim 83.

85. An immunoconjugate comprising the isolated antigen-binding protein according to any one of claims 1-80 and/or the polypeptide according to any one of claims 81-82.

86. A cell comprising and/or expressing the isolated antigen-binding protein according to any one of claims 1-80, comprising and/or expressing the polypeptide according to any one of claims 81-82, comprising the nucleic acid molecule of claim 83, comprising the vector of claim 84, and/or comprising the immunoconjugate of claim 85.

87. A pharmaceutical composition comprising the isolated antigen-binding protein according to any one of claims 1-80, the polypeptide according to any one of claims 81-82, the nucleic acid molecule of claim 83, the vector of claim 84, the immunoconjugate of claim 85, and/or the cell of claim 86, and an optional pharmaceutically acceptable vehicle.

88. A pharmaceutical combination comprising the isolated antigen-binding protein according to any one of claims 1-80, the polypeptide according to any one of claims 81-82, the nucleic acid molecule of claim 83, the vector of claim 84, the immunoconjugate of claim 85, the cell of claim 86, and/or the pharmaceutical composition of claim 87.

89. A kit comprising the isolated antigen-binding protein according to any one of claims 1-80, the polypeptide according to any one of claims 81-82, the nucleic acid molecule of claim 83, the vector of claim 84, the immunoconjugate of claim 85, the cell of claim 86, the pharmaceutical composition of claim 87, and/or the pharmaceutical combination of claim 88.

90. A kit according to claim 89 for detecting the presence and/or amount of Siglec in a sample.

91. The kit according to claim 90, wherein the Siglec includes Siglec-15.

92. A method of preparing the isolated antigen-binding protein according to any one of claims 1-80 and/or the polypeptide according to any one of claims 81-82, comprising culturing the cell of claim 86 under conditions capable of expressing the antigen-binding protein and/or the polypeptide.

93. The method of claim 92, comprising culturing the cell under conditions capable of expressing the antigen-binding protein.

94. The method of claim 92, comprising culturing the cell under conditions capable of expressing the polypeptide.

95. A method of detecting Siglec in a sample, comprising administering the isolated antigen-binding protein according to any one of claims 1-80, administering the polypeptide according to any one of claims 81-82, administering the nucleic acid molecule of claim 83, administering the vector of claim 84, administering the immunoconjugate of claim 85, administering the cell of claim 86, administering the pharmaceutical composition of claim 87, administering the pharmaceutical combination of claim 88, and/or using the kit according to any one of claims 89-91.

96. The method of claim 95, wherein the Siglec includes Siglec-15.

97. A method of affecting binding of Siglec or a functionally active fragment thereof to a ligand thereof, comprising administering the isolated antigen-binding protein according to any one of claims 1-80, administering the polypeptide according to any one of claims 81-82, administering the nucleic acid molecule of claim 83, administering the vector of claim 84, administering the immunoconjugate of claim 85, administering the cell of claim 86, administering the pharmaceutical composition of claim 87, administering the pharmaceutical combination of claim 88, and/or using the kit according to any one of claims 89-91.

98. The method of claim 97, wherein the method includes in vivo, ex vivo, and/or in vitro methods.

99. The method according to any one of claims 97-98, wherein the Siglec or a functionally active fragment thereof includes Siglec-15 or a functionally active fragment thereof.

100. The method according to any one of claims 97-99, wherein the ligand of Siglec includes Leucine-rich repeat-containing protein 4C and/or sialyl-Tn.

101. A method of affecting immune cell proliferation, comprising administering the isolated antigen-binding protein according to any one of claims 1-80, administering the polypeptide according to any one of claims 81-82, administering the nucleic acid molecule of claim 83, administering the vector of claim 84, administering the immunoconjugate of claim 85, administering the cell of claim 86, administering the pharmaceutical composition of claim 87, administering the pharmaceutical combination of claim 88, and/or using the kit according to any one of claims 89-91.

102. The method of claim 101, wherein the method includes in vivo, ex vivo, and/or in vitro methods.

103. The method according to any one of claims 101-102, wherein the affecting immune cell proliferation includes reducing the Siglec-15-associated immune cell proliferation inhibition.

104. The method according to any one of claims 101-103, wherein the immune cells include immune cells derived from human.

105. The method according to any one of claims 101-104, wherein the immune cells include lymphocytes.

106. The method according to any one of claims 101-105, wherein the immune cells include peripheral blood lymphocytes.

107. The method according to any one of claims 101-106, wherein the immune cells include T cells.

108. The method according to any one of claims 101-107, wherein the immune cells include CD4⁺ cells and/or CD8⁺ cells.

109. A method of affecting cytokine secretion, comprising administering the isolated antigen-binding protein according to any one of claims 1-80, administering the polypeptide according to any one of claims 81-82, administering the nucleic acid molecule of claim 83, administering the vector of claim 84, administering the immunoconjugate of claim 85, administering the cell of claim 86, administering the pharmaceutical composition of claim 87, administering the pharmaceutical combination of claim 88, and/or using the kit according to any one of claims 89-91.

110. The method of claim 109, wherein the secretion includes in vivo, ex vivo, and/or in vitro secretions.

111. The method according to any one of claims 109-110, wherein the cytokine secretion includes lipopolysaccharide-associated cytokine secretion.

112. The method according to any one of claims 109-111, wherein the cytokine includes an inflammatory factor.

113. The method according to any one of claims 109-112, wherein the cytokine includes TNF-α and/or IL-6.

114. Use of the isolated antigen-binding protein according to any one of claims 1-80, the polypeptide according to any one of claims 81-82, the nucleic acid molecule of claim 83, the vector of claim 84, the immunoconjugate of claim 85, the cell of claim 86, the pharmaceutical composition of claim 87, and/or the pharmaceutical combination of claim 88 in the manufacture a kit.

115. The use of claim 114, wherein the kit is used to detect the presence and/or amount of Siglec in a sample.

116. The use of claim 115, wherein the Siglec includes Siglec-15.

117. Use of the isolated antigen-binding protein according to any one of claims 1-80, the polypeptide according to any one of claims 81-82, the nucleic acid molecule of claim 83, the vector of claim 84, the immunoconjugate of claim 85, the cell of claim 86, the pharmaceutical composition of claim 87, the pharmaceutical combination of claim 88, and/or the kit according to any one of claims 89-91 in the manufacture of a medicament for the prevention and/or treatment of a disease and/or condition.

118. The use of claim 117, wherein the disease and/or condition includes a tumor.

119. The use according to any one of claims 117-118, wherein the tumor includes a Siglec-associated tumor.

120. The use of claim 119, wherein the Siglec includes Siglec-15.

121. The use according to any one of claims 117-120, wherein the disease and/or condition includes a solid tumor.

122. The use according to any one of claims 117-121, wherein the disease and/or condition includes colon cancer.
